# EUROPÄISCHE PATENTSCHRIFT

(11) **EP 1 230 354 B1**
(45) Veröffentlichungstag und Bekanntmachung des Hinweises auf die Patenterteilung: **07.01.2004**
(21) Anmeldenummer: 00979539.4
(22) Anmeldetag: 07.11.2000
(51) Int. Cl.: C12N 15/00

(54) **PERMANENTE AMNIOZYTEN-ZELLLINIE, IHRE HERSTELLUNG UND VERWENDUNG ZUR HERSTELLUNG VON GENTRANSFERVEKTOREN**
PERMANENT AMNIOCYTE CELL LINE, THE PRODUCTION THEREOF AND ITS USE FOR PRODUCING GENE TRANSFER VECTORS
LIGNEE CELLULAIRE PERMANENTE D'AMNIOCYTES, SA PRODUCTION ET SON UTILISATION POUR LA PRODUCTION DE VECTEURS DE TRANSFERT DE GENES

(30) Priorität: 18.11.1999 DE 19955558
(43) Veröffentlichungstag der Anmeldung: 14.08.2002
(73) Patentinhaber: CEVEC Pharmaceuticals GmbH, 50931 Köln (DE)
(72) Erfinder: KOCHANEK, Stefan, 50935 Köln (DE); SCHIEDNER, Gudrun, 50733 Köln (DE)
(74) Vertreter: Dehmel, Albrecht, Dr.
(86) Internationale Anmeldenummer: PCT/EP2000/010992
(87) Internationale Veröffentlichungsnummer: WO 2001/036615

(56) Entgegenhaltungen:
- WO-A-97/00326
- SCHIEDNER GUDRUN ET AL: "Efficient transformation of primary human amniocytes by E1 functions of Ad5: Generation of new cell lines for adenoviral vector production." HUMAN GENE THERAPY, Bd. 11, Nr. 15, 10. Oktober 2000 (2000-10-10), Seiten 2105-2116, XP000974022 ISSN: 1043-0342
- PLOUZEK C A ET AL: "ISOLATION AND CHARACTERIZATION OF A HUMAN AMNION EPITHELIAL CELL LINE THAT EXPRESSES THE PREGNANCY-SPECIFIC BETA-1 GLYCOPROTEIN GENE" ENDOCRINOLOGY, Bd. 129, Nr. 2, 1991, Seiten 950-958, XP000974066 ISSN: 0013-7227

## Beschreibung

Die vorliegende Erfindung betrifft eine permanente Amniozyten-Zelllinie, enthaltend mindestens eine Nukleinsäure, die die Expression der Genprodukte der E1A- und E1B-Region von Adenovirus bewirkt. Weiterhin betrifft die vorliegende Erfindung die Herstellung einer permanenten Amniozyten-Zelllinie und ihre Verwendung zur Herstellung von Gentransfervektoren. Weitere Aspekte sind die Verwendung von Amniozyten bzw. der adenoviralen Genprodukte der E1A- und E1B-Region zur Herstellung von permanenten Amniozyten-Zelllinien.

### Adenoviren

Adenoviren sind eine relativ homogene Guppe von Viren, die durch ein ikosahedisches Kapsid charakterisiert sind, das hauptsächlich aus den viral-kodierten Hexon-, Penton- und Fiber-Proteinen, sowie einem linearen, doppelsträngigen DNA-Genom mit einer Größe von etwa 36 Kilobasen (kb) besteht. Das virale Genom enthält an den Enden die Invertierten Terminalen Wiederholungssequenzen (ITRs), bei denen es sich um den viralen Replikationsursprung handelt. Ferner befindet sich am linken Ende des Genoms das Verpackungssignal, das für die Verpackung des viralen Genoms in die Viruskapside im Verlauf eines Infektionszyklus erforderlich ist. Adenoviren sind von vielen Spezies isoliert worden. Es gibt mehr als 40 verschiedene menschliche Serotypen, basierend auf zwischen den verschiedenen Serotypen diskrimierenden Parametern wie Hämagglutination, Tumorigenität und DNA-Sequenzhomologie (Wigand et al., in: Adenovirus DNA, Doerfler Hrsg., Martinus Nijoff Publishing, Boston, S. 408-441, 1986). Adenovirale Vektoren sind bisher meist von den Serotypen 2 (Ad2) und 5 (Ad5) abgeleitet. Infektionen durch Ad2 und Ad5 sind endemisch beim Menschen. Ad2 und Ad5 sind im Menschen nicht onkogen und haben eine gute Sicherheitsdokumentation, da in den USA erfolgreich und komplikationslos Vakzinierungen an militärischem Personal durchgeführt wurden (Pierce et al., Am. J. Epidemiol. 87, 237-246, 1968). Die Biologie von Adenoviren ist relativ gut verstanden, da Adenoviren in der Molekularbiologie als experimentelles Werkzeug für die Aufklärung verschiedener fundamentaler biologischer Prinzipien wie DNA-Replikation, Transkription, RNA-Spleißen und zellulärer Transformation eine wesentliche Rolle gespielt haben. Der Eintritt der adenoviralen Partikel in die Zelle im Rahmen einer Infektion erfolgt durch Rezeptor-vermittelte Endozytose, bei der nach heutiger Ansicht die Interaktion der Knob-Domäne des Fiberproteins mit dem Coxsackie-Adenovirus-Rezeptor (CAR) die Adhäsion des Viruspartikels an die Zelloberfläche vermittelt (Bergelson et al., Science 275, 1320-1323, 1997). In einem zweiten Schritt kommt es zur Intemalisierung des Viruspartikels, für die die Interaktion der Pentonbasis mit Integrinen eine wesentliche Rolle spielt (Wickham et al., Cell 73, 309-319, 1993). Nach dem Eintritt des Partikels in die Zelle gelangt das virale Genom als DNA-Proteinkomplex in den Zellkern. Der adenovirale Infektionszyklus wird in eine frühe und eine späte Phase eingeteilt, die durch den Beginn der adenoviralen Replikation getrennt werden (Shenk, in: Virology, Fields Hrsg., Lippincott-Raven Publishing, Philadelphia, S. 2111-2148, 1996). In der frühen Phase kommt es zur Expression der frühen viralen Funktionen E1, E2, E3 und E4. Die späte Phase ist charakterisiert durch die Transkription später Gene, die für die Expression viraler Strukturproteine und zur Herstellung von neuen viralen Partikeln verantwortlich sind.

E1A ist das erste virale Gen, das nach Erreichen des Zellkern durch das virale Chromosom exprimiert wird. Das E1A-Gen kodiert für die 12S- und 13S-Proteine, die durch alternatives Spleißen der E1A-RNA gebildet werden. Die E1A-Proteine aktivieren die Transkription einer Reihe von zellulären und viralen Genen durch Interaktion mit Transkriptionsfaktoren. Die Hauptfunktionen von E1A sind a) die Aktivierung der anderen frühen viralen Funktionen E1B, E2, E3 und E4 und b) die Induktion ruhender Zellen, in die S-Phase des Zellzyklus einzutreten. Die alleinige Expression von E1A führt zum programmierten Zelltod von Zellen (Apoptose).

E1B ist eines der frühen viralen Gene, die durch E1A aktiviert werden. Das E1B-Gen kodiert für das E1B-55-kD-Protein und das E1B-19-kD-Protein, die durch alternatives Spleißen der E1B-RNA entstehen. Das 55-kD-Protein moduliert die Progression des Zellzyklus durch Interaktion mit dem p53-Tumorsuppressorgen, ist involviert in die Verhinderung des Transports zellulärer mRNA spät während der Infektion, und verhindert die E1A-induzierte Apoptose von Zellen. Das E1B-19-kD-Protein ist ebenfalls für die Verhinderung der E1A-induzierten Apoptose von Zellen wichtig.

Alle humanen Adenoviren sind in der Lage, Zellen von Nagern in der Zellkultur zu transformieren. Für die onkogene Transformierung ist in der Regel die Koexpression von E1A und E1B erforderlich.

Eingebettet in die E1B-Transkriptionseinheit ist das Protein IX-Gen, das für eine Strukturkomponente des viralen Kapsids kodiert.

Die E2A- und E2B-Gene kodieren für verschiedene Proteine, die für die Replikation des viralen Genoms essentiell sind. Hierbei handelt es sich um das Vorläuferprotein des terminalen Proteins (pTP), die DNA-Polymerase (Pol) und das einzelstrangbindende Protein (SSBP). Bei der Replikation bindet pTP an die ITRs des viralen Genoms. Hier dient es als Proteinprimer für die DNA-Replikation, die durch Pol zusammen mit zellulären Faktoren initiiert wird. Für die DNA-Kettenverlängerung sind Pol, SSBP und der zelluläre Faktor NFII und vermutlich weitere Faktoren erforderlich.

E4 kodiert für verschiedene Proteine. Unter anderem blockiert das E4-34-kD-Protein, zusammen mit dem E1B-55-kD-Protein die Akkumulation von zellulären mRNAs im Zytoplasma, und gleichzeitig erleichtert es den Transport viraler RNAs vom Zellkern in das Zytoplasma.

Nach Beginn der Replikation des viralen Genoms kommt es zur Expression der viralen Strukturproteine, die für die Etablierung des viralen Kapsids und für die Komplexierung der viralen DNA mit viral-kodierten DNA-bindenden Proteinen erforderlich sind. Zunächst wird offenbar ein leeres Kapsid gebildet, in das das virale Genom im Verlauf eintritt. Für diesen Prozeß ist ein *cis*-Element auf dem viralen Genom erforderlich, das sogenannte Verpackungssignal, das am linken Ende des viralen Genoms lokalisiert ist und sich bei Ad5 über einen Bereich von Basenpaar 260 bis Basenpaar 460 erstreckt (Hearing et al., J. Virol. 61, 2555-2558, 1987; Graeble und Hearing, J. Virol. 64, 2047-2056, 1990). Das Verpackungssignal überlappt mit dem E1A-Enhancer, der für die Aktivität des EIA-Promoters essentiell ist. Der genaue Mechanismus der Verpackung des viralen Genoms in die Viruskapside ist nicht geklärt, jedoch ist es wahrscheinlich, dass hierfür die Interaktion von zellulären und/oder viralen Proteinen mit dem Verpackungssignal erforderlich ist.

### Adenovirus-Vektoren

Adenovirale Vektoren sind als Expressionsvektoren, insbesondere im Rahmen der Gentherapie, von besonderer Bedeutung. Hierfür gibt es mehrere Gründe: Die Biologie von Adenoviren ist gut untersucht. Die Viruspartikel sind stabil und können relativ einfach und mit hohen Titern produziert werden. Das adenovirale Genom ist genetisch leicht manipulierbar. Adenovirus-Vektoren können replizierende und nicht-replizierende Zellen in vitro und in vivo effizient transduzieren.

### a) Adenovirale Vektoren der ersten Generation

Adenovirale Vektoren der ersten Generation (Gilardi et al., FEBS Letters 267, 60-62, 1990; Stratford-Perricaudet et al., Hum. Gene Ther. 1, 241-256, 1990) sind durch Deletionen der E1A- und E1B-Gene charakterisiert. E1A und E1B haben transformierende und transaktivierende Eigenschaften. Ferner ist E1A für die Aktivierung viraler Gene und E1B für die Akkumulation viraler Transkripte erforderlich. In einigen Vektoren ist außerdem E3 deletiert, um die Kapazität für die Aufnahme fremder DNA zu erhöhen. E3 ist für die Herstellung von Adenoviren in der Zellkultur entbehrlich. Die Kapazität für die Aufnahme fremder DNA beträgt etwa 8 kb. Adenovirus-Vektoren der ersten Generation sind bisher überwiegend in 293-Zellen produziert worden (siehe unten), die den E1A- und E1B-Defekt der Vektoren komplementieren.

### b) Adenovirale Vektoren der zweiten Generation

Adenovirale Vektoren der zweiten Generation sind durch Deletionen von E2 und/oder E4 zusätzlich zu Deletionen von E1A und E1B charakterisiert (Engelhardt et al., Proc. Natl. Acad. Sci., USA 91, 6196-6200, 1994; Yang et al., Nature Genet., 7, 362-367, 1994; Gorziglia et al., J.Virol. 70, 4173-4178,1996; Krougliak und Graham, Hum. Gene Ther. 6, 1575-1586, 1995; Zhou et al., J. Virol. 70, 7030-7038, 1996). In einigen Vektoren ist außerdem E3 deletiert, um die Kapazität für die Aufnahme fremder DNA zu erhöhen. Adenovirale Vektoren der zweiten Generation wurden entwickelt, um die Transkription von viralen Genen und die Expression viraler Proteine weiter zu reduzieren und um somit die antivirale Immunantwort weiter zu verringern. Die Kapazität für die Aufnahme fremder DNA ist gegenüber adenoviralen Vektoren der ersten Generation nicht wesentlich erhöht. Adenovirus-Vektoren der zweiten Generation werden in Zelllinien produziert, die zusätzlich zu E1A und E1B den jeweiligen Defekt (E2 und/oder E4) komplementieren.

### c) Adenovirale Vektoren großer DNA-Kapazität

Adenovirale Vektoren großer DNA-Kapazität sind dadurch charakterisiert, dass sie keine viralen kodierenden DNA-Sequenzen enthalten (Kochanek et al., Proc. Natl. Acad. Sci. U.S.A. 93, 5731-5736, 1996; Fisher et al., Virology 217, 11-22, 1996; Kumar-Singh und Chamberlain, Hum. Mol. Genet. 5, 913-921, 1996).

Diese Vektoren enthalten lediglich die viralen Enden unter Einschluß der ITRs und des Verpackungssignals. Die Kapazität für die Aufnahme fremder DNA beträgt etwa 37 kb, da der weit überwiegende Teil des adenoviralen Genoms entfernt ist. Es sind verschiedene Systeme zur Herstellung adenoviraler Vektoren großer DNA-Kapazität beschrieben worden (Kochanek et al., *supra*; Parks et al., Proc. Natl. Acad. Sci. USA 93, 13565-13570, 1996; Hardy et al., J. Virol. 71, 1842-1849, 1997). Der Vorteil dieser adenoviralen Vektoren mit großer DNA-Kapazität im Vergleich zu adenoviralen Vektoren der ersten und zweiten Generation liegt in der größeren Kapazität für die Aufnahme fremder DNA und in einer geringeren Toxizität und Immunogenität (Schiedner et al., Nature Genet. 18, 180-183, 1998; Morral et al., Hum. Gene Ther. 9, 2709-2716, 1998). Derzeit werden adenovirale Vektoren großer Kapazität mit Hilfe eines E1A- und E1B-deletierten Helfervirus produziert, das die viralen Funktionen, die für einen produktiven Infektionszyklus erforderlich sind, in trans zur Verfügung stellt. Bisher sind adenovirale Vektoren großer DNA-Kapazität in 293-Zellen oder in von 293-Zellen abgeleiteten Zelllinien hergestellt worden. In einer. der Herstellungsweisen (Parks et al., *supra*; Hardy et al., *supra*) werden adenovirale Vektoren in modifizierten 293-Zellen produziert, die zusätzlich zu E1A und E1B die Rekombinase Cre des Bakteriophagen P1 exprimieren. In diesem System wird das Verpackungssignal des Helfervirus von loxP-Erkennungssequenzen des Bakteriophagen P1 flankiert. Bei Infektion von Cre-exprimierenden 293-Zellen mit Helfervirus und dem adenoviralen Vektor großer DNA-Kapazität wird das Verpackungssignals des Helfervirus exzidiert. Aus diesem Grund wird überwiegend der ein normales Verpackungssignal enthaltende Vektor, nicht jedoch das Helfervirus verpackt.

### d) Deletierte adenovirale Vektoren

Diese Vektoren wurden als Vektoren der ersten Generation beschrieben, die loxP-Erkennungssequenzen des Bakteriophagen P1 derart im viralen Genom positioniert haben, dass bei Infektion von Cre-exprimierenden 293-Zellen durch Rekombination zwischen den loxP-Erkennungssequenzen der größte Teil der viralen kodierenden Sequenzen oder die viralen kodierenden Sequenzen insgesamt entfernt werden. Die Genomgröße dieser Vektoren beträgt etwa 9 kb. Die Kapazität für die Aufnahme fremder DNA liegt ebenfalls bei etwa 9 kb (Lieber et al., J. Virol. 70, 8944-8960, 1996).

### Adeno-Assoziiertes Virus (AAV)

AAV gehört zur Familie der Parvoviren, Genus Dependoviren, und hat zwei verschiedene Lebensformen, entweder es tritt als lytisches Virus oder als Provirus auf Für einen lytischen Infektionsablauf erfordert das Virus eine Koinfektion mit einem Helfervirus (Adenovirus, Vacciniavirus, Herpes simplex-Virus). Bei Abwesenheit eines Helfervirus kann AAV nicht replizieren, integriert in das Genom und existiert dort als inaktives Provirus. Bei Infektion von Zellen, die AAV als integriertes Provirus tragen, z.B. mit Adenovirus, kann das Provirus wieder in einen lytischen Infektionszyklus eintreten (Samulski, Curr. Opin. Genet. Dev. 3, 74-80, 1993).

AAV-Kapside enthalten ein einzelsträngiges, lineares DNA-Genom mit entweder positiver oder negativer Polarität. Es existieren mehrere AAV-Serotypen. Der am besten untersuchte Serotyp ist AAV-2. Das Genom von AAV-2 besteht aus 4680 Nukleotiden. An den Enden enthält das Genom Invertierte Terminale Wiederholungssequenzen (ITRs), die eine Länge von 145 Basenpaaren haben. Die ersten 125 Basenpaare bilden eine T-förmige Haamadelstruktur, die aus zwei internen Palindromen besteht.

Das AAV-Genom kodiert für nicht-strukturelle Replikations (Rep)-Proteine und für strukturelle Kapsid (Cap)-Proteine. Die verschiedenen Replikationsproteine (Rep78, Rep68, Rep52, Rep40) werden durch Verwendung verschiedener Promoteren (p5 und p19) sowie durch alternatives Spleißen erzeugt. Die verschiedenen Kapsidproteine (VP1, VP2, VP3) werden durch alternatives Spleißen unter Verwendung des p40 Promoters erzeugt.

### AAV-Vektoren

AAV-Vektoren enthalten lediglich die ITRs von AAV und einige benachbarte, nichtkodierende AAV-Sequenzen. Aus diesem Grund beträgt die Kapazität für die Aufnahme fremder DNA etwa 4,5 kb. Es sind verschiedene Systeme zur Herstellung der rekombinanten AAV-Vektoren beschrieben worden (Skulimowski und Samulski, in: Methods in Molecular Genetics, Bd. 7, Adolph Hrsg., Academic Press, S. 3-12). In diesen Systemen werden diejenigen Komponenten zur Verfügung gestellt, die für Replikation, Expression und Verpackung des rekombinanten Vektors erforderlich sind. Im Einzelnen handelt es sich um Expressionskassetten, die für die Rep- und Cap-Proteine von AAV kodieren sowie die adenoviralen Helferfunktionen. Bei den für die AAV-Herstellung erforderlichen adenoviralen Helferfunktionen handelt es sich im Einzelnen um E1A, E1B, E2, E4 und VA. Die E1A- und E1B-Funktionen werden in den 293-Zellen, die bisher für die Herstellung verwendet werden, zur Verfügung gestellt. In den bisher beschriebenen Herstellungsverfahren werden die E2-, E4- und VA-Funktionen derzeit meist entweder durch Koinfektion mit Adenovirus oder durch Kotransfektion mit E2-, E4- und VA-exprimierenden Plasmiden zur Verfügung gestellt (Samulski et al., J. Virol. 63, 3822-3828, 1989; Allen et al., J. Virol. 71, 6816-6822, 1997; Tamayose et al., Hum. Gene Ther. 7, 507-513, 1996; Flotte et al., Gene Ther. 2, 29-37, 1995; Conway et al, J. Virol. 71, 8780-8789, 1997; Chiorini et al., Hum. Gene Ther. 6, 1531-1541, 1995; Ferrari et al., J. Virol. 70, 3227-3234, 1996; Salvetti et al., Hum. Gene Ther. 9, 695-706, 1998; Xiao et al., J. Virol. 72, 2224-2232, 1998, Grimm et al., Hum. Gene Ther. 9, 2745-2760, 1998; Zhang et al., Hum. Gene Ther. 10, 2527-2537, 1999). Alternativ wurden Strategien entwickelt, in denen für die Herstellung von AAV-Vektoren Adenovirus/AAV- oder Herpes simplex-Virus/AAV-Hybridvektoren verwendet wurden (Conway et al., *supra*; Johnston et al., Hum. Gene Ther. 8, 359-370, 1997, Thrasher et al., Gene Ther. 2, 481-485, 1995; Fisher et al., Hum. Gene Ther. 7, 2079-2087, 1996; Johnston et al., Hum. Gene Ther. 8, 359-370, 1997). Allen diesen Verfahren ist gemeinsam, dass derzeit E1A- und E1B-exprimierende 293-Zellen zur Herstellung verwendet werden.

### Herstellungs-Zelllinien

Aus Sicherheitsgründen haben in der Regel adenovirale Vektoren, die für den Einsatz beim Menschen gedacht sind, Deletionen der E1A- und E1B-Gene. Die Herstellung erfolgt in komplementierenden Zelllinien, die die E1-Funktionen in *trans* zur Verfügung stellen. Die meisten adenoviralen Vektoren sind bisher in der 293-Zelllinie produziert worden. In den letzten Jahren sind weitere Zelllinien hergestellt worden, die für die Herstellung von E1-deletierten adenoviralen Vektoren verwendet werden können.

### a) HEK-293-Zellen

HEK-293-Zellen waren lange Zeit die einzigen Zellen, die für die Herstellung von E1-deletierten adenoviralen Vektoren verwendet werden konnten. HEK-293-Zellen wurden 1977 durch Transfektion von gescherter adenoviraler DNA in menschliche embryonale Nierenzellen (HEK-Zellen) hergestellt. Bei insgesamt acht Transfektionsexperimenten mit durchschnittlich jeweils 20 HEK-Kulturen konnte lediglich ein einzelner immortalisierter Zellklon gewonnen werden (Graham et al., J. Gen. Virol. 36, 59-74, 1977). Die aus diesem Zellklon etablierte Zelllinie (HEK-293-Zellen) enthalten die vollständigen linken 11% des adenoviralen Genoms (Basenpaar 1 bis 4344 des Ad5-Genoms) einschließlich der E1A- und E1B-Gene sowie des linken ITRs und des adenoviralen Verpackungssignals (Louis et al., Virology 233, 423-429, 1997). Ein wesentliches Problem für die Herstellung adenoviraler Vektoren ist die Sequenzhomologie zwischen E1-deletierten adenoviralen Vektoren und dem in 293-Zellen integrierten Anteil adenoviraler DNA. Homologe Rekombination zwischen dem Vektorgenom und der in 293-Zellen integrierten adenoviralen DNA ist für die Entstehung von replikationskompetenten Adenoviren (RCA) verantwortlich (Lochmüller et al., Hum. Gene Ther. 5, 1485-1491, 1994; Hehir et al., J. Virol. 70, 8459-8467, 1996). HEK-293-Zellen sind aus diesem Grund für die Herstellung adenoviraler Vektoren in pharmazeutischer Qualität nicht geeignet, da Herstellungseinheiten häufig mit unakzeptablen Mengen an RCA kontaminiert sind. RCA ist in für klinische Anwendung hergestellten Produkten nicht akzeptabel, da replikationskompetente Adenoviren eine deutlich höhere Toxizität als replikationsdefiziente Adenoviren haben, unkontrolliert in menschlichen Geweben replizieren können und ferner replikationsdefekte Adenoviren komplementieren können (Lochmüller et al., *supra*; Imler et al., Hum. Gene Ther. 6, 711-721, 1995; Hehir et al., *supra*)

### b) Humane Embryonale Retinazellen (HER-Zellen) und etablierte Zelllinien

Obwohl Zellen von Nagern leicht mit adenoviralen E1-Funktionen transformiert werden können, haben sich primäre menschliche Zellen als relativ refraktär für eine Transformation mit E1A und E1B erwiesen. Wie oben erwähnt, konnte Graham und Mitarbeiter lediglich einen einzelnen Zellklon aus HEK-Zellen isolieren, die mit gescherter Ad5-DNA transfiziert worden waren. Gallimore und Mitarbeiter versuchten über lange Zeit erfolglos, primäre HEK-Zellen mit E1-Funktionen von Ad12 zu transformieren (Gallimore et al., Anticancer Res., 6, 499-508, 1986). Diese Experimente wurden erfolglos über einen Zeitraum von drei Jahren mit mehr als 1 mg des EcoRI-C DNA-Fragmentes von Ad12 durchgeführt, das die E1A- und E1B-Gene enthielt. Nach vielen Versuchen konnten trotz einer großen Zahl an durchgeführten Experimenten lediglich vier Ad12-E1-HEK-Zelllinien isoliert werden (Whittaker et al., Mol. Cell. Biol., 4, 110-116, 1984). Ebenso versuchten Gallimore und Mitarbeiter erfolglos, andere primäre menschliche Zellen mit E1-Funktionen zu transformieren, einschließlich Keratinozyten, Hautfibroblasten, Hepatozyten und Urothelzellen (Gallimore et al., Anticancer Res., 6, 499-508, 1986). Bei dem einzigen menschlichen Zelltyp, der bisher reproduzierbar mit adenoviralen E1-Funktionen transformiert werden konnte, handelt es sich um menschliche embryonale Retinazellen (HER-Zellen). HER-Zellen sind eine Mischung von Zellen, die aus der weißen neuralen Retina stammen. Zur Gewinnung dieser Zellen muß ein Auge eines menschlichen Fetus, üblicherweise zwischen der 16. und 20. Schwangerschaftswoche, aus der Augenhöhle entfernt werden. Das Auge wird mit einem horizontalen Schnitt eröffnet und die weiße neurale Retina kann mit einer Pinzette entfernt und in Zellkultur genommen werden.

Basierend auf früheren Beobachtungen, dass a) Ad12-induzierte Tumoren in erster Linie von primitivem neuralen Epithel abstammen (Mukai et al., Prog. Neuropathol. 3, 89-128, 1976), und dass b) Ad12 nach intraokularer Inokulation retinale Tumoren in Ratten und Pavianen induziert (Mukai et al., *supra*; Mukai et al., Science 210, 1023-1025, 1980), fanden Byrd und Mitarbeiter, dass menschliche embryonale Retinoblasten (HER-Zellen) mit den E1-Genen von Ad12 transformiert werden können (Byrd et al., Nature 298, 69-71, 1982). Obwohl die Effizienz der Transformation von HER-Zellen geringer als die von primären Rattenzellen waren, war die Effizienz der Transformation mehr als 100-fach höher als die von HEK-Zellen. Diese Untersuchungen wurden initiiert, um komplementierende Zelllinien herzustellen, mir deren Hilfe Ad12-E1-Mutanten isoliert werden konnten.

In weiteren Untersuchungen dieser Arbeitsgruppe (Gallimore et al., Cancer Cells 4, 339-348, 1986) konnte gezeigt werden, dass HER-Zellen mit Plasmid-DNA, die die E1A- und E1B-Gene von Ad5 exprimiert, effizient transformiert werden können. Die Effizienz der Transformation und die Etablierung E1A und E1B exprimierender Zelllinien war mit den E1-Genen von Ad5 etwa 20-fach höher als mit E1-Genen von Ad12.

Basierend auf diesen Daten etablierten Fallaux und Mitarbeiter (Fallaux et al., Hum. Gene Ther. 7, 215-222, 1996; Fallaux et al., Hum. Gene Ther. 9, 1909-1917, 1998) E1A und E1B exprimierende Zelllinien durch Transformation von HER-Zellen mit Plasmiden, die die E1A- und E1B-Gene von Ad5 exprimierten. Die Zelllinie 911 wurde durch Transformation mit einem Plasmid hergestellt, das die E1A- und E1B-Gene von Ad5 enthält (Nukleotide 79-5789 des Ad5-Genoms) und E1A unter Kontrolle des natürlichen E1A-Promoters exprimiert (Fallaux et al., *supra*; Patentanmeldung WO97/00326). Weitere E1A und E1B exprimierende HER-Zelllinien konnten durch Transfektion eines Plasmids etabliert werden, das die Nukleotide 459-3510 des Ad5-Genoms enthält, bei dem das E1A-Gen unter Kontrolle des humanen Phosphoglycerat-Kinase(PGK)-Promoters steht, und bei dem das natürliche E1B-Polyadenylierungssignal durch die poly(A)-Sequenz des Hepatitis B-Virus(HBV)-Oberflächenantigens ersetzt ist (Fallaux et al., *supra*; Patentanmeldung WO 97/00326). Diese HER-Zelllinien wurden als PER-Zelllinien bezeichnet. Der Vorteil dieser neueren PER-Zelllinien im Vergleich zu 293-Zellen oder der 911-Zelllinie liegt in der fehlenden Sequenzhomologie zwischen der DNA von adenoviralen Vektoren der ersten Generation und der integrierten Ad5-DNA. Aus diesem Grund ist die Möglichkeit der Entstehung von RCA deutlich reduziert. Diese E1A- und E1B- transformierten HER-Zelllinien (911-Zellen und PER-Zellen) konnten den E1-Defekt von adenoviralen Vektoren der ersten Generation komplementieren und somit für die Herstellung dieser Vektoren verwendet werden.

In ähnlicher Weise wird in einer Publikation von Imler und Mitarbeitern eine Zelllinie erwähnt, die durch Transformation von HER-Zellen mit Plasmid pTG6559 etabliert wurde (Imler et al., *supra*; s.a. WO 94/28152). Plasmid pTG6559 enthält die kodierenden Sequenzen der E1A-, E1B-Gene und des Protein IX-Gens (Nukleotide 505-4034 des Genoms von Ad5), wobei das E1A-Gen unter Kontrolle des Phosphoglycerat-Kinase(PGK)-Promoters der Maus steht und das gemeinsame Polyadenylierungssignal der E1B- und Protein IX-Gene durch das Polyadenylierungssignal des β-Globin-Gens des Kaninchens ersetzt war.

Im Gegensatz zu den beschriebenen Versuchen, primäre menschliche Zellen durch Transformation mit den E1A- und E1B-Genen von Ad5 zu etablieren, wurde in einigen Fällen versucht, E1A und E1B von verschiedenenen Serotypen stabil in bereits etablierten Zelllinien zu exprimieren (Grodzicker et al., Cell, 21, 453-463, 1980; Babiss et al., J. Virol. 46, 454-465, 1983; Shiroki et al., J. Virol. 45, 1074-1082, 1983; Imler et al., *supra*; s.a. WO 94/28152). Nachteile dieser Zelllinien ist die Notwendigkeit der Koexpression eines Selektionsmarkers und die häufig mangelnde Stabilität der Expression von E1A und E1B. Da es sich bei diesen Zelllinien bereits von vorne herein um immortalisierte Zelllinien handelt, ist die Expression von E1A und E1B für das Überleben der Zelllinien nicht erforderlich, so dass die natürliche Selektion durch E1A und E1B in diesem Fall und im Gegensatz zur Verwendung von primären Zellen entfällt.

In der Vergangenheit war die Herstellung von Zelllinien zur Herstellung von adenoviralen Vektoren oder zur Herstellung von AAV-Vektoren mit besonderen Schwierigkeiten verbunden. Humane embryonale Nierenzellen (HEK-Zellen) können aus menschlichen Feten aus der Niere gewonnen werden. Hierzu wird eine Niere aus einem Fetus entfernt und Nierenzellen werden in Zellkultur genommen. Durch Transfektion von HEK-Zellen mit gescherter Ad5-DNA und Integration des linken Endes der Ad5-DNA sowie Expression der E1A- und E1B-Gene kam es in einem einzigen publizierten Fall zur Transformation dieser Zellen. Eine einzige Zelllinie ( 293-Zellen) konnte so etabliert werden (Graham et al., *supra*; s.o. "Herstellungszelllinien", Abschnitt a). 293-Zellen werden für die Herstellung von adenoviralen Vektoren und für die Herstellung von AAV-Vektoren verwendet.

Humane embryonale Retinazellen (HER-Zellen) können aus dem Augapfel menschlicher Feten gewonnen werden. Hierzu wird ein Auge des Fetus entfernt und aus der Netzhaut stammende Zellen werden in Kultur genommen. Durch Transfektion von HER-Zellen mit den adenoviralen E1A- und E1B-Genen konnten HER-Zellen transformiert werden (s.o. "Herstellungszelllinien", Abschnitt b). Mit E1A und E1B transformierte Zellen können zur Herstellung von adenoviralen Vektoren verwendet werden.

In beiden Fällen ist es nötig, aus menschlichen Feten, die entweder aus einem spontanen oder therapeutischen Abort oder aus einem Schwangerschaftsabbruch aus sozialer Indikation stammen, ein Organ zu entnehmen und von diesem Organ eine Zellkultur zu etablieren. Nach Etablierung einer Primärkultur können diese Zellen dann durch Transfektion mit den adenoviralen E1A- und E1B-Genen transformiert werden. So etablierte Zelllinien, die E1A und E1B exprimieren, können dann für die Herstellung von adenoviralen Vektoren oder AAV-Vektoren verwendet werden.

Es ist offensichtlich, dass die Gewinnung von primären Zellen aus Organen von Feten aufwendig ist. Da nur aus frischem Gewebe eine Primärkultur etabliert werden kann, sind besondere logistische Anstrengungen zu unternehmen, um an geeignetes Gewebe zu gelangen. Außerdem macht die Verwendung fetalen Gewebes, das entweder von einem Spontanabort, einem therapeutischen Abort oder aus einem Schwangerschaftsabbruch aus sozialer Indikation stammt, für die Etablierung der Primärkultur besondere ethische Überlegungen und Sorgsamkeit erforderlich. Obwohl das Labor der Erfinder in einer Frauenklinik angesiedelt ist, in der häufig Schwangerschaftsabbrüche durchgeführt werden, war es nicht möglich, über einen Zeitraum von mehr als einem Jahr an geeignetes Gewebe zu gelangen. Eine Entnahme von fetalem Gewebe nach dem Abort erfordert eine Einverständniserklärung nach entsprechender Aufklärung der Schwangeren. Nach detailierter Aufklärung über das Vorhaben, i.e. die Entfernung eines Auges des Feten für medizinisch-wissenschaftliche Untersuchungen, war es mehrfach nicht möglich, ein Einverständnis der Schwangeren für den Eingriff mit Organentnahme zu erlangen.

Die Verwendung einer permanenten Amniozyten-Zelllinie zur Herstellung von Gentransfervektoren ist bisher nicht beschrieben worden. Es wurde lediglich über menschliche Amniozyten berichtet, die mit dem Simian Virus (SV40) und/oder dem Kirsten-Sarcoma-Virus transformiert worden sind (Sack, In Vitro 17 S.1-19, 1981; Walen, et al., In Vitro Cell Dev. Biol. 22, 57-65, 1986). Eine Infektion mit SV40 allein verlieh eine verlängerte Lebensdauer (Immortalisierung genannt), während eine Infektion mit dem Kirsten-Sarcoma-Virus allein die Lebensspanne nicht verlängerte. Eine Infektion mit beiden Viren führte schließlich zu einer malignen Tumorzelle (Walen und Amstein, *supra*). Hierbei ist zu beachten, daß SV40-transformierte Amniozyten-Zellinien nicht zur Produktion von Gentransfervektoren geeignet sind, da diese Zellen selber SV40, bekanntermaßen ein onkogenes Virus, produzieren (Graffney et al., Cancer Res. 30, 871-879, 1970). Die Transformierbarkeit von menschlichen Zellen mit SV40 läßt außerdem keine Aussagen über die Transformierbarkeit mit den E1-Funktionen von Adenovirus und deren Verwendung zur Produktion/Herstellung von Gentransfervektoren zu. Beispielsweise können Keratinozyten mit SV40 transformiert werden (siehe Sack, *supra*), jedoch können Keratinozyten wie auch Hautfibroblasten und Hepatozyten offenbar nicht mit Ad12 transformiert werden (Gallimore et al., 1986, *supra*). In Hinsicht auf die Herstellung von viralen Vektoren, insbesondere adenoviralen Vektoren, in immortalisierten Zellen ist zudem nicht allein die Immortalisierbarkeit mit den jeweiligen Immortalisierungsfunktionen, sondern auch eine gute Infizierbarkeit und ein guter produktiver Infektionsablauf von Bedeutung. Diese Eigenschaften sind nicht vorauszusagen; die Frage, ob ein bestimmter Zelltyp für die Herstellung von Gentransfervektoren zu verwenden ist, muß für jeden Zelltyp neu bestimmt werden.

Aufgabe der vorliegenden Erfindung war es daher, ein neues Verfahren zur effizienten, einfachen und leicht reproduzierbaren Herstellung einer Amniozyten-Zelllinie zur Verfügung zu stellen, und deren Verwendung unter anderem zur Herstellung von adenoviralen Vektoren, von AAV-Vektoren und von retroviralen bzw. lentiviralen Vektoren.

Es wurde völlig überraschend gefunden, dass die Transfektion von Fruchtwasserzellen (Amniozyten), die routinemässig aus diagnostischen Gründen im Rahmen der Pränataldiagnostik durch Fruchtwasserpunktion (Amniozentese) gewonnen werden, mit den adenoviralen E1A- und E1B-Genen zu einer großen Zahl von permanenten Zelllinien führte, die die E1A- und E1B-Gene funktionell aktiv exprimieren und die für die Herstellung von Gentransfervektoren geeignet sind.

Ein Gegenstand der vorliegenden Erfindung ist daher eine permanente Amniozyten-Zelllinie, enthaltend mindestens eine Nukleinsäure, die die Expression der Genprodukte der E1A- und E1B-Region von Adenovirus bewirkt. Unter einer "permanenten Zelllinie" versteht man gemäß der vorliegenden Erfindung, dass die entsprechenden Zellen in irgendeiner Weise derart genetisch verändert sind, dass sie permanent in Zellkultur weiterwachsen können. Demgegenüber versteht man unter "primären Zellen" Zellen, die durch Entnahme aus einem Organismus und Subkultur erhalten wurden und nur eine begrenzte Lebensdauer zeigen. Eine permanente Amniozyten-Zelllinie im Sinne der vorliegenden Erfindung kann durch das hierin vorgeschlagene Verfahren, das die Transfektion von primären Amniozyten mit den E1-Funktionen von Adenovirus beinhaltet, erhalten werden. Bei der mindestens einen Nukleinsäure, die die Expression der E1-Genprodukte von Adenovirus bewirkt, kann es sich um jede geeignete Nukleinsäure bzw. Nukleinsäuren handeln, die zu einer stabilen Expression dieser Genprodukte führen. Sie kann/können in das Genom der Zelle integriert, d.h. chromosomal, oder extrachromosomal, z.B. als episomal replizierendes Plasmid oder Mini-Chromosom, vorliegen. Die Expression der verschiedenen Genprodukte kann dabei von ein und demselben Nukleinsäuremolekül oder beispielsweise verschiedenen Nukleinsäuremolekülen bewirkt werden. Als "Expression" wird im Stand der Technik der Prozeß des Herstellens des Produkts eines Gens, das entweder ein spezifisches Protein ist, das ein spezifisches Merkmal oder eine spezifische Eigenschaft bewirkt, oder von RNA-Formen verstanden, die nicht in Proteine translatiert werden (z.B. antisense RNAs, tRNAs). Dem Fachmann werden angesichts der vorliegenden Beschreibung, insbesondere auch des vorgeschlagenen Verfahrens, geeignete Möglichkeiten zur Erzielung der gewünschten Expression ersichtlich sein. Die erfindungsgemäße Amniozyten-Zelllinie eignet sich neben ihrer Verwendung zur Herstellung von Gentransfervektoren im allgemeinen insbesondere zur Herstellung von adenoviralen Vektoren der ersten Generation, die durch Deletionen der E1A- und E1B-Gene charakterisiert sind, die durch die Zelllinie komplementiert werden.

Vorzugsweise bewirkt die mindestens eine Nukleinsäure weiterhin die Expression der Genprodukte der E2A-, E2B- und/oder E4-Region von Adenovirus und/oder der Cre-Rekombinase. Hierdurch eignet sich die Zelllinie insbesondere zur Herstellung von adenoviralen Vektoren der zweiten Generation, die durch Deletionen von E2- und/oder E4-Genen zusätzlich zu den Deletionen der E1A- und E1B-Gene charakterisiert sind. Die Expression der Cre-Rekombinase des Bakteriophagen P1 ist insbesondere bei der Herstellung von adenoviralen Vektoren großer Kapazität mit Hilfe eines E1A- und E1B-deletierten Helfervirus von Vorteil (siehe auch Parks et al., *supra*; Hardy et al., *supra*). Vorteilhafterweise steht die Expression der Genprodukte der E1A-Region unter der Kontrolle eines konstitutiven Promoters, vorzugsweise des Phosphoglycerat-Kinase (PGK)-Promoters. Für die Expression der Genprodukte der E1B-Region ist es von Vorteil, wenn sie unter der Kontrolle eines adenoviralen Promoters, vorzugsweise des adenoviralen E1B-Promoters steht. Alternativ dazu kann zum Beispiel auch ein Promoter des Zytomegalie-Virus (CMV) eingesetzt werden. Vorzugsweise stammen alle adenoviralen Genprodukte von einem Adenovirus des gleichen Subgenus, z.B. des Adenovirus Typ 5 (Ad5) des Menschen. Üblicherweise handelt es sich bei der permanenten Amniozyten-Zelllinie um eine humane Zelllinie, da diese besonders zur Herstellung von Gentransfervektoren geeignet ist, die sich von menschlichen Viren, wie z.B. einem humanen Adenovirus oder einem humanen AAV, ableiten.

Alternativ dazu kann es sich auch um eine Zelllinie von Primaten oder anderen Säugetieren wie z.B. dem Rind handeln, die sich insbesondere zur Herstellung von Gentransfervektoren eignen, die von in der jeweiligen Spezies vorkommenden und endemischen Viren abgeleitet sind. Zum Beispiel eignen sich zur Herstellung von Vektoren, die von einem bovinen Adenovirus abgeleitet sind, permanente Amniozyten-Zelllinien, die durch Transformation von Amniozyten mit den EIA- und EIB-Genen eines bovinen Adenovirus gewonnen wurden.

Ein weiterer Gegenstand der vorliegenden Erfindung ist ein Verfahren zur Herstellung einer permanenten Amniozyten-Zelllinie, insbesondere einer wie vorangehend definierten Amniozyten-Zelllinie, gekennzeichnet durch das Transfizieren von Amniozyten mit mindestens einer Nukleinsäure, die die Expression der adenoviralen Genprodukte der E1A-Region und E1B-Region bewirkt. Die erhaltenen Zellklone können dann gegebenenfalls weiter isoliert und unter Erhalt von Einzelzelllinien, wenn gewünscht, kloniert werden. Unter dem Begriff "Transfizieren" wird hierin jedes Verfahren verstanden, dass sich zum Einbringen der genannten Nukleinsäure(n) in die Zellen eignet. Als Beispiele sind die Elektroporation, liposomale Systeme jeglicher Art und Kombinationen dieser Verfahren zu nennen. Unter dem Begriff "Amniozyten" werden hierin im weiteren Sinne alle Zellen verstanden, die im Fruchtwasser vorhanden sind und durch Fruchtwasserpunktion gewonnen werden können. Sie stammen entweder vom Amnion oder von fetalem Gewebe, das im Kontakt mit der Amnionflüssigkeit ist. Es wurden drei Hauptklassen von Amniozyten beschrieben, die aufgrund morphologischer Kriterien unterschieden werden: Fibroblastenartige Zellen (F-Zellen), epitheloide Zellen (E-Zellen) und Amnionflüssigkeitszellen (amniotic fluid cells; AF-Zellen) (Hohn et al., Pediat. Res. 8, 746-754, 1974). AF-Zellen sind der vorherrschende Zelltyp. Im engeren Sinne werden hierin unter "Amniozyten" daher Amniozyten vom AF-Typ verstanden. Vorzugsweise werden primäre Amniozyten verwendet. Als "primäre" Zellen werden Zellen bezeichnet, die durch Entnahme aus einem Organismus und Subkultur erhalten werden können und nur eine begrenzte Lebensdauer zeigen, während sogenannte "permanente" Zelllinien unbegrenzt weiterwachsen können. Besonders bevorzugt ist hierbei die Verwendung von humanen primären Amniozyten, die zur Herstellung von humanen Zelllinien führen (s.o.). Jedoch können auch primäre Amniozyten von Primaten und weiteren Säugetierspezies wie vom Rind Verwendung finden. Dem Fachmann wird angesichts der vorliegenden Beschreibung auch ersichtlich sein, dass analog Zellen, die aus den Amnionmembranen, zum Beispiel durch Trypsinieren, oder durch eine Chorionzottenbiopsie erhalten werden können, zur Herstellung von entsprechenden permanenten Zelllinien verwendet werden können.

Bei der mindestens einen Nukleinsäure, die die Expression der adenoviralen E1-Genprodukte bewirkt, kann es sich um genomische DNA, cDNA, synthetische DNA, RNA und mRNA handeln. Vorzugsweise wird die Nukleinsäure in Form eines DNA-Expressionsvektors verwendet. Beispiele hierfür sind integrative Vektoren, bakterielle Plasmide, episomal replizierende Plasmide oder Mini-Chromosomen. Bevorzugt sind Expressionsplasmide, die durch Transfektion in das Genom der Empfängerzelle zur Integration gebracht werden. Mit dem Ausdruck "mindestens eine Nukleinsäure" wird zum Ausdruck gebracht, dass die die Expression bewirkenden Elemente sich sowohl auf ein und dergleichen Nukleinsäure als auch auf verschiedenen Nukleinsäuren befinden können. Beispielsweise können getrennte Nukleinsäuren für die Expression der Genprodukte der E1A-, E1B-, E2A-, E2B- und/oder E4-Region und/oder der Cre-Rekombinase vorgesehen sein. Es ist auch denkbar, dass die zu transfizierenden Amniozyten bereits eines dieser Genprodukte exprimieren, so dass nur die Expression des weiteren bzw. der weiteren Genprodukte zu bewirken ist, oder dass die Expression eines oder mehrerer dieser Genprodukte lediglich durch das Einbringen von geeigneten Regulatorelementen angeschaltet wird. Dem Fachmann stehen geeignete Techniken und Verfahren zur Herstellung und ggf. Mutagenese von Nukleinsäuren sowie zur Genexpression und Proteinanalyse zur Verfügung (s. z.B. Sambrook, J. et al., Molecular Cloning: A Laboratory Manual, Cold Spring Harbor Laboratory Press (1989); Glover, D.M., DNA cloning: A practical approach, Bd. II: Expression Systems, IRL Press (1995); Ausubel et al., Short protocols in molecular biology, John Wiley & Sons (1999); Rees, A.R. et al., Protein engineering: A practical approach, IRL press (1993)). Vorzugsweise wird das Genprodukt bzw. die Genprodukte der E1A-Region unter der Kontrolle eines konstitutiven Promoters, insbesondere des Phosphoglycerat-Kinase (PGK)-Promoters und die Genprodukte der E1B-Region unter der Kontrolle eines adenoviralen Promoters, insbesondere des adenoviralen E1B-Promoters exprimiert. Anstelle des adenoviralen Promoters kann beispielsweise auch ein Promoter des Zytomegalie-Virus (CMV) verwendet werden.

In einer besonderen Ausführungsform wird durch Transfizieren der Amniozyten und/oder der erhaltenen Zelllinie zusätzlich die Expression der erhaltenen Genprodukte E2A- und/oder E2B- und/oder E4-Region von Adenovirus und/oder der Cre-Rekombinase bewirkt. Hierbei stehen dem Fachmann sämtliche Möglichkeiten, die vorangehend diskutiert oder auf sonstige Weise aus dem Stand der Technik bekannt sind, zur Verfügung. Hinsichtlich der einzelnen Gene wird zusätzlich auf folgende Angaben verwiesen: E2A: Genbank Acc. #M73260; Kruiyer et al., Nucl. Acids Res. 9, 4439-4457, 1981; Kruiyer et al., Nucl. Acids Res. 10, 4493-4500, 1982. E2B: Genbank Acc. #M73260; Dekker et al. Gene 27, 115-120, 1984; Shu et al., Virology 165, 348-356, 1988. E3: Genbank Acc. #M73260; Cladaras et al., Virology 140, 28-43, 1985. E4: Genbank Acc. #M73260 und D12587; Virtanen et al., J. Virol. 51, 822-831, 1984; Dix et al., J. Gen. Virol. 73, 2975-2976, 1992. Die Leseraster sind teilweise nur für Ad2 bekannt und können dann in der Regel durch Sequenzvergleich bei z.B. Ad5 zugeordnet werden. Cre-Rekombinase: Genbank Acc. #X03453; Sternberg et al., J. Mol. Biol. 187, 197-212, 1986.

Vorzugsweise stammen die genannten adenoviralen Genprodukte alle von einem bestimmten adenoviralen Serotyp, insbesondere von dem humanen Adenovirus Typ 5 (Ad5). Der jeweilige adenovirale Serotyp, von dem die E1A- und E1B-Gene stammen, die zur Transformation von Amniozyten verwendet werden, ist nicht kritisch für diese Erfindung. Beispiel adenoviraler Serotypen, die in der vorliegenden Erfindung angewandt werden können, sind im Stand der Technik bekannt und schließen mehr als 40 humane Serotypen ein, z.B. Ad12 (Subgenus A), Ad3 und Ad7 (Subgenus B), Ad2 und Ad5 (Subgenus C), Ad8 (Subgenus D), Ad4 (Subgenus E), Ad40 (Subgenus F) (Wigand et al, in: Adenovirus DNA, Doerfler, Hrsg., Martinus Nijhoff Publishing, Boston, S. 408-441, 1986). In einer bevorzugten Ausführungsform dieser Erfindung werden zur Herstellung von adenoviralen Vektoren, die vom Subgenus C abgeleitet sind, Amniozyten mit E1A- und E1B-Genen transformiert, die von einem Adenovirus des gleichen Subgenus stammen. Zum Beispiel werden zur Herstellung von adenoviralen Vektoren des Serotyps 2 oder 5 Amniozyten mit den E1A- und E1B-Genen des Serotyps 2 oder 5 transformiert. Zur Herstellung von adenoviralen Vektoren, die auf Ad12 basieren, werden Amniozyten mit den E1A- und E1B-Genen von Ad12 transformiert usw.. Zur Herstellung von nicht-human-adenoviralen Vektoren, einschließlich der von den gut bekannten Adenoviren, die vom Rind, Schaf, Schwein und anderen Säugern abstammen, werden für die Herstellung Amniozyten-Zelllinien durch Transformation von Amniozyten der jeweiligen Spezies hergestellt. Dies ist in der Regel erforderlich, da adenovirale Funktionen in der Regel über Speziesgrenzen hinweg nicht effizient komplementiert werden können.

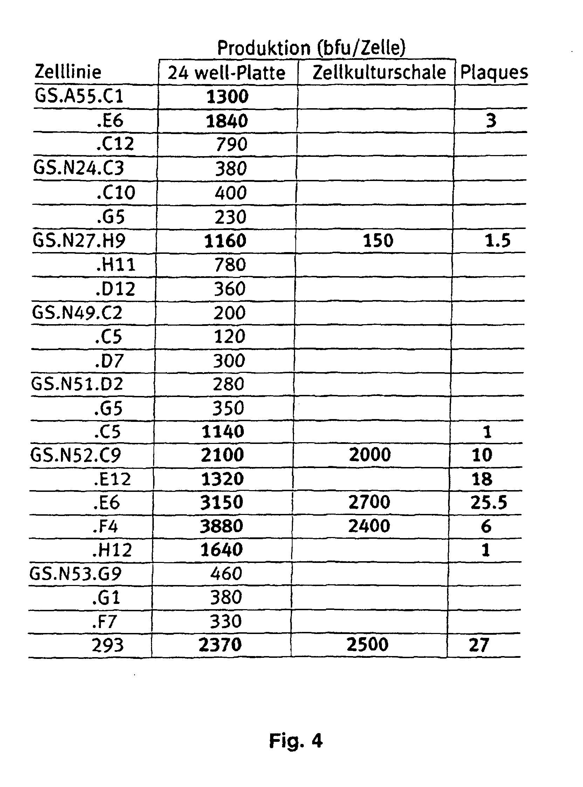

In einer besonderen Ausführungsform der Erfindung wurden Amniozyten, die aus diagnostischen Gründen im Rahmen der Pränataldiagnostik durch Fruchtwasserpunktion gewonnen und für diagnostische Zwecke nicht mehr gebraucht wurden, mit einem Expressionsplasmid transfiziert, das die E1A- und E1B-Gene von Ad5 exprimierte. Dieses Konstrukt war so konzipiert, dass das E1A-Gen unter Kontrolle des Phosphoglycerat-Kinase-Promoters der Maus, und das E1B-Gen unter Kontrolle des natürlichen adenoviralen E1B-Promoters stand. Die natürliche E1B-Spleißakzeptorstelle sowie die EIB-Polyadenylierungssequenz wurden durch entsprechende Sequenzen des SV40-Virus ersetzt. Wenige Wochen nach Transfektion mit der Plasmid-DNA wurden zahlreiche Zellklone beobachtet, die isoliert, kloniert, als immortalisierte Zelllinien etabliert und analysiert wurden. Alle analysierten Zellklone exprimierten die E1A- und E1B-Proteine. Durch Infektion mit einem E1-deletierten, β-gal exprimierenden adenoviralen Vektor und anschließende Färbung konnte gezeigt werden, daß alle Zellen infizierbar waren. Infektionsexperimente mit E1-deletierten adenoviralen Vektoren der ersten Generation ergaben, dass die Zelllinien zur Herstellung von adenoviralen Vektoren geeignet sind. In diesen Experimenten wurden die Zelllinien zunächst mit einem β-gal exprimierenden adenoviralen Vektor der ersten Generation infiziert. Nach 48-72 Stunden, wenn die Zellen einen zytopathischen Effekt (CPE) zeigten, wurden die Zellen geerntet und der adenovirale Vektor wurde durch dreimaliges Einfrieren und Auftauen aus den Zellen befreit. Mit einem Teil des Zelllysates wurden 293-Zellen infiziert und die β-gal-Expression wurde histochemisch etwa 24 Stunden nach dem Gentransfer nachgewiesen. Aus der Zahl an β-gal positiven Zellen konnte direkt die Ausbeute des Vektors durch Produktion in den einzelnen Zelllinien berechnet werden. So lassen sich ohne Schwierigkeiten und reproduzierbar Amniozyten-Zelllinien gewinnen, die für die Herstellung von Gentransfervektoren sehr gut geeignet sind. Einige der isolierten Zelllinien erlaubten eine genauso gute oder bessere Herstellung von adenoviralen Vektoren als 293-Zellen. Wie zu erwarten wiesen die Zelllinien Unterschiede in der Produktion an rekombinantem Adenovirusvektor auf (siehe Fig. 4). Die Zelllinien N52.E6 und N52.F4 zeichneten sich durch rasches Wachstum und besonders gute Produktion von adenoviralen Vektoren aus, günstige Eigenschaften für die Verwendung dieser Zelllinien zur Herstellung von Gentransfervektoren.

Das Design des E1A und E1B exprimierenden Expressionsplasmids, das für die Transformation der Amniozyten verwendet wurde, schließt die Entstehung von replikationskompetenten Adenoviren (RCA) durch homologe Rekombination eines adenoviralen Vektors oder eines adenoviralen Helfervirus mit der in den transformierten Amniozyten integrierten DNA im Gegensatz zu 293-Zellen aus. Alternativ dazu können die einzelnen E1-Funktionen auf verschiedenen Expressionsplasmiden in die zu transfizierenden Zellen eingebracht werden. Natürlich ist es auch möglich, wie bei der 293-Zelllinie eine Transformation von Amniozyten vorzunehmen und die bei der Herstellung von Gentransfervektoren erzeugten Chargen z.B. mittels eines PCR- oder eines Infektions-Assays auf den Gehalt von RCA zu testen. Die RCA enthaltenden Chargen können dann ggf. verworfen werden.

Somit betrifft ein weiterer Gegenstand der vorliegenden Erfindung eine permanente Amniozyten-Zelllinie, die gemäss dem hierin vorgeschlagenen Verfahren erhalten werden kann. In einer spezifischen Ausführungsform betrifft die Erfindung die permanente Amniozyten-Zelllinie N52.E6, die am 26.10.1999 bei der Deutschen Sammlung von Mikroorganismen und Zellkulturen GmbH (DSMZ) gemäß dem Budapester Vertrag hinterlegt wurde und die Hinterlegungsnummer DSM ACC2416 erhalten hat.

In einem weiteren Aspekt betrifft die vorliegende Erfindung die Verwendung von Amniozyten zur Herstellung von Adenovirus-transformierten permanenten Amniozyten-Zelllinien. Unter dem Begriff "Adenovirus-transformiert" wird hierin eine Transformation durch eines oder mehrere transformierende Adenovirus-Gene verstanden. Als "Transformation" wird in diesem Zusammenhang die Umwandlung einer eukaryontischen Zelle, die einer Wachstumskontrolle unterliegt, in eine unbegrenzt wachsende, sogenannte permanente Zelllinie bezeichnet. Ein weiterer Aspekt ist die Verwendung der adenoviralen Genprodukte der E1A- und E1B-Region zur Herstellung von permanenten Amniozyten-Zelllinien.

Die vorliegende Erfindung umfaßt als Gegenstand weiterhin die Verwendung einer permanenten Amniozyten-Zelllinie zur Herstellung von Gentransfervektoren. Unter "Gentransfervektoren" werden hierin allgemein alle Vektoren verstanden, mit denen ein oder mehrere therapeutische Gene in die gewünschten Zielzellen übertragen bzw. eingeführt werden können und insbesondere virale Vektoren, die diese Eigenschaft besitzen. Desweiteren können die permanenten Amniozyten-Zellinien für die Herstellung von Adenovirusmutanten verwendet werden. Unter "Adenovirusmutanten" werden Adenoviren verstanden, die mindestens eine Mutation in den E1A- und/oder E1B-Genen aufweisen. In einer bevorzugten Ausführungsform tragen sie im Gegensatz zu adenoviralen Gentransfervektoren jedoch keine therapeutische Gene. Ein typisches Beispiel hierfür sind Adenovirusmutanten, in denen das E1B-55-kD-Protein nicht exprimiert ist (z.B. die Adenovirusmutante *dl*1520 (Barker et al., Virology 156, 107-121, 1987)). Adenovirusmutanten, in denen das E1B-55-kD-Protein nicht exprimiert wird, sind für die Therapie von Tumorerkrankungen von großem Interesse, da es zu einer Vermehrung der Virusmutante ausschließlich in Tumorzellen und nicht oder in nicht nennenswertem Ausmaß in primären normalen Zellen kommt (Bischoff et al., Science 274, 373-376, 1996; Kim et al., Nature Med. 4, 1341-1342, 1998).

Eine bevorzugte Ausführungsform ist die Verwendung einer permanenten Amniozyten-Zelllinie zur Herstellung von Adenovirusvektoren, AAV (Adenoassoziiertes Virus)-Vektoren, Retrovirusvektoren, Lentivirusvektoren, chimären Adenovirus-AAV-Vektoren, chimären Adenovirus-Retrovirusvektoren und/oder chimären Adenovirus-Lentivirusvektoren. Weiterhin ist auch eine Verwendung zur Herstellung von Herpesvektoren möglich.

AAV-Vektoren enthalten üblicherweise lediglich die ITRs von AAV und einige benachbarte, nichtkodierende AAV-Sequenzen. Ihre Kapazität für die Aufnahme fremder DNA beträgt etwa 4,5 kb. Wie oben beschrieben existieren verschiedene Systeme zur Herstellung der rekombinanten AAV-Vektoren. Allen diesen Systemen ist gemein, dass diejenigen Komponenten zur Verfügung gestellt werden, die für Replikation, Expression und Verpackung des rekombinanten Vektors erforderlich sind. Im Einzelnen handelt es sich um Expressionskassetten, die für die AAV-Rep- und -Cap-Proteine kodieren sowie die adenoviralen Helferfunktionen. Bei den für die AAV-Herstellung erforderlichen adenoviralen Helferfunktionen handelt es sich um die E1A-, E1B-, E2-, E4- und VA-Gene. Die E1A- und E1B-Funktion werden in den E1A und E1B exprimierenden Amniozyten-Zelllinien zur Verfügung gestellt und können deshalb für die Herstellung von AAV-Vektoren verwendet werden. Die E2-, E4- und VA-Funktionen können durch Koinfektion mit Adenovirus oder durch Kotransfektion mit E2, E4 und VA exprimierenden Plasmiden bzw. über die Verwendung von Adenovirus/AAV- oder Herpes simplex-Virus/AAV-Hybridvektoren zur Verfügung gestellt werden. (Samulski et al., *supra*; Allen et al., *supra*; Tamayose et al., *supra*; Flotte et al., *supra*; Conway et al., *supra*; Chiorini et al., *supra*; Ferrari et al., *supra*; Salvetti et al., *supra*; Xiao et al., *supra*; Grimm et al., *supra*; Zhang et al., *supra*).

Retrovirusvektoren, d.h. von Retroviren abgeleitete Vektoren, sind ebenfalls als Vehikel zur Transfektion im Rahmen von gentherapeutischen Maßnahmen von großer Bedeutung, zum Beispiel für eine Gentherapie im Zentralnervensystem (Suhr et al., Arch. Neurol. 56, 287-292, 1999). Retrovirale Vektoren können in stabilen Vektor-Produktions-Zelllininen hergestellt werden oder durch eine transiente Transfektion. Die einzelnen Komponenten, die zur Herstelllung von retroviralen Vektoren verwendet werden, schließen üblicherweise eine oder mehrere Plasmide ein, die die Struktur- und die Replikations- und Integrationproteine exprimieren und ferner ein Plasmid, das den Vektor selbst enthält (Miller, in:. Retroviruses, Coffin, Hughes, Varmus Hrsg., Cold Spring Harbor Laboratory Press, 1997, S. 437-473). Werden Plasmide verwendet, die einen Replikationsursprung enthalten, wie z.B. den Replikationsursprung von SV40, werden die Amniozyten-Zelllinien derart modifiziert, dass Proteine, die die Replikation des Plasmids fördern, stabil exprimiert werden. Zum Beispiel wird im Falle von Plasmiden, die den SV40-Replikationsursprung enthalten, eine Amniozyten-Zelllinie verwendet, die das T-Antigen von SV40 exprimiert.

Lentivirusvektoren sind von Lentiviren abgeleitete Vektoren (Naldini et al., Science 272, 263-267, 1996; Dull et al., J. Virol. 72, 8463-8471, 1998). Lentivirale Vektoren können in stabilen Vektor-Produktions-Zelllininen hergestellt werden oder durch eine transiente Transfektion.

Unter "chimären Vektoren" werden Vektoren verstanden, die das Produkt einer Fusion von Nukleinsäuren zweier oder mehrerer verschiedener viraler Vektoren sind. Für die Herstellung von chimären Vektoren können gemäß der vorliegenden Beschreibung permanente Amniozyten-Zelllinien verwendet werden. In diesem System trägt z.B. ein Adenovirusvektor, bevorzugt ein Adenovirusvektor großer Kapazität, ein DNA-Fragment, das die Sequenzinformation für ein integrierendes Virus, das z.B. von einem Retrovirus oder von AAV abgeleitet ist. Nach Transduktion einer Zielzelle wird das integrierende Virus, das ein therapeutisches Gen trägt, vom adenoviralen Hintergrund freigesetzt (z.B. im Falle eines retroviralen Inserts durch Herstellung infektiöser retroviraler Partikel, die benachbarte Zellen transduzieren und stabil als DNA integrieren). Beispiele von chimären Vektoren, die in 293-Zellen produziert wurden, sind in der Vergangenheit beschrieben worden, z.B. als chimäre Adenovirus-Retrovirus-Vektoren (Feng et al., Nature Biotech. 15, 866-870, 1997) und als chimäre Adenovirus-AAV-Vektoren (Recchia et al., Proc. Natl. Acad. Sci. USA 96, 2615-2620, 1999). Die Herstellung in E1A und E1B exprimierenden Aminozyten-Zelllinien ist zu bevorzugen, da es im Gegensatz zu 293-Zellen nicht zur Entstehung replikationskompetenter Vektoren durch homologe Rekombination kommen kann.

Adenovirusvektoren, d.h. von Adenoviren abgeleitete Vektoren, sind insbesondere als Vehikel zur Transfektion im Rahmen von gentherapeutischen Maßnahmen von großer Bedeutung. Bei den Adenovirusvektoren kann es sich um Adenovirusvektoren der ersten Generation, Adenovirusvektoren der zweiten Generation, Adenovirusvektoren grosser DNA-Kapazität und/oder deletierte Adenovirusvektoren handeln, die mit Hilfe einer permanenten Amniozyten-Zelllinie hergestellt werden.

### a) Herstellung von adenoviralen Vektoren der ersten Generation

Adenovirale Vektoren der ersten Generation sind in der Regel durch Deletionen der E 1A- und E1B-Gene charakterisiert. Teilweise enthalten adenovirale Vektoren der ersten Generation zusätzlich zur Deletion der E1A- und E1B-Gene auch Deletionen der E3-Region. E3-Funktionen sind für das Wachstum von adenoviralen Vektoren in der Zellkultur entbehrlich.

Adenovirale Vektoren der ersten Generation können in E1A und E1B exprimierenden Amniozyten-Zelllinien erzeugt werden. Hierzu werden die E1A und E1B exprimierenden Zellen bevorzugterweise mit 3-5 infektiösen Einheiten pro Zelle (3-5 MOI) infiziert. Nach etwa 36 bis 72 Stunden zeigen die Zellen einen zytopathischen Effekt. Die Zellen werden nach Standardprotokollen geerntet. Adenoviraler Vektor kann aus diesen durch CsCI-Dichtegradienten-Zentrifugation oder durch chromatographische Verfahren aufgereinigt werden.

### b) Herstellung von adenoviralen Vektoren der zweiten Generation

Adenovirale Vektoren der zweiten Generation sind durch Deletionen der E1Aund E1B-Gene charakterisiert. Teilweise enthalten adenovirale Vektoren der zweiten Generation auch eine Deletion der E3-Region. Zusätzlich zur Deletion der E1A- und E1B-Gene sind adenovirale Vektoren der zweiten Generation durch eine Inaktivierung und vorzugsweise Deletion von mindestens einem weiteren essentiellen adenoviralen Gen, z.B. einem E2A-Gen, einem E2B-Gen und/oder einem E4-Gen, charakterisiert, oder beispielsweise durch Deletionen von E2-Funktionen in Kombination mit Deletionen von E4-Funktionen.

Für die Herstellung von adenoviralen Vektoren der zweiten Generation müssen diejenigen Funktionen, die der Vektor selber durch Inaktivierung und/oder Deletion nicht exprimiert, von der Amniozyten-Zelllinie zur Verfügung gestellt werden. Hierzu können E1A und E1B stabil exprimierende Amniozyten-Zelllinien durch Transfektion von Expressionskassetten, die die für ein oder mehrere weitere adenovirale Funktionen kodierenden Genprodukte exprimieren, stabil modifiziert werden. Zum Beispiel wird für die Herstellung eines adenoviralen Vektors der zweiten Generation, der zusätzlich zur Deletion der E1A- und E1B-Gene auch eine Deletion eines E2A-, E2B- und/oder E4-Gens aufweist, das entsprechende Gen bzw. die entsprechenden Gene durch Transfektion zusammen mit einem Selektionsmarker in die E1A und E1B exprimierende Amniozyten-Zelllinie eingeführt. Zellklone, die zusätzlich zur Expression von E1A- und E1B-Funktionen auch E2A-, E2B- bzw. E4-Funktionen exprimieren, können dann für die Herstellung des jeweiligen Vektors der zweiten Generation verwendet werden. In der Regel stehen die E2- und/oder E4-Gene unter der transkriptionellen Kontrolle eines heterologen Promoters, der entweder konstitutiv aktiv oder regulierbar ist.

### c) Herstellung von adenoviralen Vektoren großer DNA-Kapazität.

Adenovirale Vektoren großer DNA-Kapazität sind dadurch charakterisiert, dass die meisten oder alle viralen kodierenden Sequenzen entfernt sind. Bevorzugterweise enthalten diese Vektoren lediglich die viralen ITRs und das virale Verpackungssignal. Die adenoviralen Funktionen werden von einem Helfervirus in *trans* zur Verfügung gestellt. Verschiedene Systeme zur Herstellung von adenoviralen Vektoren großer DNA-Kapazität wurden beschrieben. Allen bisher beschriebenen Systemen, die ein Helfervirus verwenden, ist gemeinsam, dass das Helfervirus einem replikationsdefekten, E1A- und E1B-deletierten Adenovirus entspricht. Das Helfervirus enthält entweder ein vollständiges Verpackungssignal (Mitani et al., Proc. Natl. Acad. Sci. USA 92, 3854-3858, 1995; Fisher et al., Virology 217, 11-22, 1996; Kumar-Singh und Chamberlain, Hum. Mol. Genet. 5, 913-921, 1996) oder ein mutiertes Verpackungssignal (Kochanek et al., Proc. Natl. Acad. Sci . U.S.A. 93, 5731-5736, 1996). In letzterem Fall wird der Vektor bevorzugt in virale Kapside verpackt, da das Helfervirus ein attenuiertes Verpackungssignal enthält und deshalb weniger effektiv verpackt wird. Alternativ kann das Verpackungssignal des Helfervirus nach der Infektion der Herstellungszelllinie durch Verwendung einer Rekombinase exzidiert werden (Parks et al., Proc. Natl. Acad. Sci. USA 93, 13565-13570, 1996; Hardy et al., J. Virol. 71, 1842-1849, 1997). Zum Beispiel kann das Verpackungssignal des Helfervirus mit loxP-Erkennungssequenzen des Bakteriophagen P1 flankiert werden. Durch Expression der Cre-Recombinase des Bakteriophagen P1 kommt es zur Exzision des Verpackungssignals des Helfervirus. Wegen des Fehlens des Verpackungssignals kommt es jedoch nicht zur Verpackung des Helfervirus in Kapside. Das Cre-Rekombinase-Gen des Bakteriophagen P1 wird durch Transfektion zusammen mit einem Selektionsmarker in die E1A und E1B exprimierende Amniozyten-Zelllinie eingeführt. Zellklone, die zusätzlich zur Expression von E1A- und E1B-Funktionen auch die Cre-Funktion des Bakteriophagen P1 exprimieren, können dann für die Herstellung des jeweiligen Vektors großer DNA-Kapazität verwendet werden.

### d) Herstellung von "deletlerten" adenoviralen Vektoren

"Deletierte" adenovirale Vektoren wurden als Vektoren der ersten Generation beschrieben, die loxP-Erkennungssequenzen des Bakteriophagen P1 derart im viralen Genom positioniert haben, dass bei Infektion von Cre-exprimierenden 293-Zellen durch Rekombination zwischen den loxP-Erkennungssequenzen der größte Teil der viralen kodierenden Sequenzen oder die viralen kodierenden Sequenzen insgesamt entfernt werden. Die Genomgröße dieser Vektoren beträgt etwa 9 kb. Die Kapazität für die Aufnahme fremder DNA liegt ebenfalls bei etwa 9 kb (Lieber et al., J. Virol. 70, 8944-8960, 1996). Zur Verwendung in der Herstellung von deletierten adenoviralen Vektoren wird das Cre-Rekombinase-Gen des Bakteriophagen P1 durch Transfektion zusammen mit einem Selektionsmarker in die E1A und E1B exprimierende Amniozyten-Zelllinie eingeführt. Zellklone, die zusätzlich zur Expression von E1A- und E1B-Funktionen auch die Cre-Funktion des Bakteriophagen P1 exprimieren, können dann für die Herstellung des jeweiligen deletierte Ad-Vektors verwendet werden.

### e) Herstellung von Tropismus modifizierten Gentransfervektoren

In einer bevorzugten Ausführungsform wird die permanente Amniozyten-Zelllinie zur Herstellung von Tropismus-modifizierten Gentransfervektoren verwendet. Der Tropismus eines Virus und eines von diesem Virus abgeleiteten viralen Vektors entscheidet, ob ein bestimmter Zelltyp mit einem Vektor erfolgreich transduziert werden kann oder nicht. Die Aufnahme eines Gentransfervektors in eine Zelle hinein ist der erste Schritt für einen erfolgreichen Gentransfer in diese Zelle. Der Tropismus eines viralen Vektors ist also für die Effizienz des *in vitro*- oder *in vivo*-Gentransfers in eine bestimmte Zelle oder in ein Gewebe ein wesentlicher Faktor. Für die Aufnahme in eine bestimmte Zelle ist die Interaktion der Oberfläche eines viralen Vektors (des Kapsids im Falle adenoviraler oder AAV-Vektoren, der Virushülle im Falle retroviraler oder lentiviraler Vektoren) mit der Zellmembran einer Zielzelle erforderlich. Obwohl der genaue Mechanismus der Aufnahme eines viralen Vektors in eine Zielzelle bei verschiedenen Vektoren teilweise unterschiedlich ist, spielt jedoch in allen Fällen die Interaktion von Oberflächenstrukturen des viralen Vektors (in der Regel Proteinliganden) mit Strukturen auf der Zielzelle (in der Regel Rezeptoren oder Adhäsionsmoleküle) eine wesentliche Rolle. Die Aufnahme von adenoviralen Vektoren erfolgt z.B. durch Rezeptor-vermittelte Endozytose. Hierbei binden Teile des adenoviralen Kapsids mit zellulären Rezeptoren. Im Falle von Ad2 oder Ad5 abgeleiteten adenoviralen Vektoren bindet nach derzeitigem Kenntnisstand in der Regel ein Teil der Knob-Domäne des Fiberproteins mit dem Coxsackie-Adenovirus-Rezeptor (CAR) und ein Teil der Pentonbasis mit αvβ3- oder αvβ5-Integrinen. Die Bindung der Knob-Domäne an CAR ist nach derzeitigem Kenntnisstand für die Adhäsion des Vektors an die Zellmembran der Zielzelle erforderlich, während die Bindung der Pentonbasis an Integrine für die Intemalisierung des Vektors in die Zielzelle erforderlich ist.

Amniozyten-Zelllinien können für die Herstellung von Tropismus-modifizierten Vektoren verwendet werden. Dies gilt z.B. für die Herstellung von adenoviralen Vektoren der ersten und zweiten Generation, für adenovirale Vektoren grosser DNA-Kapazität, für deletierte adenovirale Vektoren, für chimäre adenovirale Vektoren, für AAV-Vektoren, für retrovirale und/oder lentivirale Vektoren. Verschiedene Strategien können zur Herstellung von Tropismus-modifizierten Vektoren in Amniozyten-Zelllinien verwendet werden. Die Strategie, die für die jeweilige Tropismusmodifikation angewandt wird, kann bei verschiedenen Vektoren (z.B. adenoviraler Vektor, AAV-Vektor, retroviraler Vektor) unterschiedlich sein. Gemeinsam ist den verschiedenen Strategien, dass die Oberfläche des jeweiligen Vektors (Viruskapsid bei adenoviralen und AAV-Vektoren, Virushülle bei retroviralen und lentiviralen Vektoren) derart verändert wird, dass die Bindung des Vektors an die Zielzelle verändert wird. Beispiele für Modifikationen für adenovirale Vektoren sind:
a) Austausch von Fiberproteinen zwischen verschiedenen Serotypen: Hierdurch entstehen adenovirale Vektoren, deren Kapsid ein Fiberprotein eines anderen Serotyps trägt. Beispiele hierfür sind der Austausch des natürlichen Fiberproteins von adenoviralen Vektoren, die vom Serotyp 2 abgeleitet sind, durch ein Fiberprotein, das vom Serotyp 17 (Zabner et al., J. Virol. 73, 8689-8695, 1999) oder vom Serotyp 9 (Roelvink et al., J. Virol. 70, 7614-7621, 1996) abgeleitet ist. Andere Beispiele sind der Austausch des natürlichen Fiberproteins von adenoviralen Vektoren, die vom Serotyp 5 abgeleitet sind, durch ein Fiberprotein, das vom Serotyp 7a (Gall et al., J. Virol. 70, 2116-2123, 1996) oder vom Serotyp 3 (Stevenson et al., J. Virol. 71, 4782-4790, 1997; Krasnykh et al., J. Virol. 70, 6839-6846, 1996; Douglas et al., Neuromuscul. Disord. 7, 284-298, 1997) abgeleitet ist.
b) Entfernung des Fiberproteins: Das Fiberprotein kann durch gentechnische Verfahren entfernt werden, so dass die Aufnahme des Vektors allein über die Interaktion der Pentonbasis oder des Hexonproteins erfolgt (Falgout et al., J. Virol. 62, 622-625, 1988; Legrand et al., J. Virol. 73, 907-919, 1999).
c) Modifikation des C-Terminus des Fiberproteins mit einem Peptid: Beispiel hierfür sind die Modifikation des C-Terminus mit einem Polylysin-Peptid (Yoshida et al., Hum. Gene Ther. 9, 2503-2515, 1998; Wickham et al., Nat. Biotechnol. 14, 1570-1573, 1996; Wickham et al., J. Virol. 71, 8221-8229, 1997), einem Polyhistidin-Peptid (Douglas et al., Nat. Biotechnol. 17, 470-475, 1999) oder einem Gastrin-Releasing-Peptid (Michael et al., Gene Ther. 2, 660-668, 1995).
d) Modifikation von Teilen der Knob-Domäne des Fiberproteins durch Insertion eines Peptids: Beispiele hierfür sind die Insertion eines FLAG-Epitops (Krasnykh et al., J. Virol. 72, 1844-1852, 1998) oder die Insertion eines RGD-Peptids (Dmitriev et al., J. Virol. 72, 9706-9713, 1998; Kasono et al., Clin Cancer Res. 5, 2571-2579, 1999).
e) Modifikation der Pentonbasis: Ein Beispiel hierfür ist der Ersatz eines RGD-Motivs innerhalb der Pentonbasis durch ein LDV-Motiv, mit dem Ziel, eine Bindung des Vektors an α4β1-Integrine zu vermitteln (Wickham et al., Gene Ther. 2, 750-756, 1995).
f) Modifikation des Hexon-Proteins: Ein Beispiel hierfür ist die Insertion eines von Poliovirus Typ 3 stammenden Epitops (Crompton et al., J. Gen. Virol. 75,133-139,1994).

Eine alternative Strategie, die zur Veränderung des Tropismus von in Amniozyten-Zelllinien hergestellten Vektoren angewandt werden kann, beruht auf der Verwendung von Liganden, die eine Bindung des Vektors an Zellmembranstrukturen, wie z.B. zellulären Rezeptoren oder Adhäsionsmoleküle, vermitteln. Diese Liganden können Peptide, Proteine oder auch Antikörper sein. Die Liganden können über verschiedene Verfahren mit der Oberfläche der Vektoren verbunden werden. Die Verbindung der Liganden mit der Oberfläche der Vektoren (der Kapside im Falle von adenoviralen oder AAV-Vektoren) kann über die Verwendung von Antikörpern oder über eine chemische Reaktion durch Crosslinking (Quervemetzung) hergestellt werden. Bei der Verwendung von Antikörpern können Antikörper verwendet werden, deren Spezifität gegen das Kapsid des Vektors (z.B. gegen die Knob-Domäne des Fiber-Proteins) gerichtet ist. Alternativ können Antikörper verwendet werden, deren Spezifität gegen ein Epitop gerichtet ist, das als Neoepitop (z.B. ein FLAG-Epitop oder ein myc-Epitop) in das Kapsid des Vektors eingebracht worden ist. Beispiele hierfür sind dem Fachmann wohlbekannt. Beispiele für die Verwendung von bispezifischen Antikörpern sind beschrieben in Wickham et al., J. Virol. 70, 6831-6838, 1996 (anti-FLAG/anti-α-Integrin); in Wickham et al., Cancer Immunol. Immunther. 45, 149-151, 1997; Harari et al., Gene Ther. 6, 801-807, 1999 (anti-FLAG/anti-E-Selectin) zur Transduktion von Endothelzellen; in Miller et al., Cancer Res. 58, 5738-5748, 1998; Blackwell et al., Arch. Otolaryngol. Head Neck Surg. 125, 856-863, 1999 (anti-Ad/anti-EGFR) zur Transduktion von Tumorzellen; in Wickham et al., J. Virol. 71, 7663-7669, 1997 (anti-FLAG/anti-CD3) zur Transduktion von T-Zellen; in Tillman et al., J. Immunol. 162, 6378-6383, 1999 (anti-CD40/anti-Ad) zur Transduktion von Dendritischen Zellen. Beispiele für die Verwendung von Einzelketten-Antikörpern mit Spezifität für eine Determinante des Viruskapsids, die an einen Liganden gekoppelt ist, sind beschrieben in Watkins et al., Gene Ther. 4, 1004-1012, 1997; in Goldman et al., Cancer Res. 57, 1447-1451, 1997; Rancourt et al., Clin. Cancer Res. 4, 2455-2461, 1998; Gu et al., Cancer Res. 59, 2608-2614, 1999; Rogers et al., Gene Ther. 4, 1387-1392, 1997 (anti-Ad/FGF2) zur Transduktion von FGF2-Rezeptor exprimierenden Tumorzellen; in Douglas et al., Nat. Biotechnol. 14, 1574-1578, 1996; Douglas et al., Neuromuscular Disord. 7, 284-298, 1997 (anti-Ad/Folat) zur Transduktion von Tumorzellen, die den Folsäure-Rezeptor auf der Zelloberfläche exprimieren.

Bei Gentransfervektoren, bei denen der natürliche Tropismus aufgehoben ist und durch einen anderen Tropismus ersetzt ist, beispielsweise durch das Einbringen eines Liganden in die Knobdomäne des Fiberproteins von Ad5, kann es notwendig sein, eine permanente Amniozyten-Zelllinie durch die vorzugsweise stabile Expression eines Rezeptors, der diesen neuen Liganden erkennt, zu modifizieren (Douglas et al., Nat. Biotechnol. 17, 470-475, 1999). Ebenso kann die permanente Amniozyten-Zelllinie für die Herstellung von Gentransfervektoren verwendet werden, die einen Defekt in der Bildung eines oder mehrerer Strukturproteine haben. Hierzu wird der jeweilige Defekt des Gentransfervektors in der permanenten Amniozyten-Zelllinie komplementiert. Zum Beispiel kann ein adenoviraler Vektor, der eine Mutation im für das Fiberprotein kodierenden Gen hat, in einer Amniozyten-Zelllinie produziert werden, die den Defekt des Fiberproteins komplementiert. Dies wird durch Einbringen einer Fiberexpressionskassette in die Amniozyten-Zelllinie und die stabile oder induzierbare Expression des Fiberproteins in dieser Amniozyten-Zelllinie erreicht (Von Seggem et al., J. Gen. Virol. 79, 1461-1468, 1998). Bei dem in der Amniozyten-Zelllinie exprimierten Fiberprotein kann es sich um ein natürliches, unmodifiziertes Fiberprotein handeln oder auch um ein verändertes, zum Beispiel Tropismus-modifiziertes Fiberprotein (Von Seggem et al., *supra*). Auch die Herstellung von adenoviralen Vektoren, denen das Fiberprotein vollkommen fehlt, kann in der permanenten Amniozyten-Zelllinie durchgeführt werden (Legrand et al., J. Virol., 73, 907-919, 1999; Von Seggem et al., J. Virol. 73, 1601-1608, 1999).

Die Verwendung von E1A und E1B exprimierenden Amniozyten-Zelllinien ist zu bevorzugen, da es im Gegensatz zu 293-Zellen nicht zur Entstehung replikationskompetenter Vektoren durch homologe Rekombination kommen kann. In einer besonderen Ausführungsform des Aspektes der Verwendung einer Amniozyten-Zelllinie zur Herstellung von Gentransfervektoren handelt es sich bei dieser Zelllinie um die erfindungsgemäße Zelllinie.

### Therapeutische Gene

Bei den Genen, insbesondere den therapeutischen Genen, die von in transformierten Amnionzellen, d.h. einer permanenten Amniozyten-Zelllinie, produzierten Vektoren kodiert und exprimiert werden können, kann es sich z. B. um jede beliebigen Muskelproteine, Gerinnungsfaktoren, Membranproteine oder Zellzyklusproteine handeln. Beispiele für Proteine, die von in transformierten Amniozyten hergestellten Vektoren exprimiert werden können, sind Dystrophin (Hoffman et al., Cell 51, 919, 1987, Faktor VIII (Wion et al., Nature 317, 726 1985, Zystische Fibrose Transmembran Regulator Protein (CFTR) (Anderson et al., Science 251, 679, 1991, Omithin-Transcarbamylase (OTC) (Murakami et al., J. Biol. Chem., 263, 18437, 1988, Alphal-Antitrypsin (Fagerhol et al., in: Hum. Genet., Bd. 11, Harris Hrsg., Plenum, New York, S.1, 1981. Die Gene, die für Proteine kodieren, sind bekannt und können aus genomischen oder cDNA-Banken kloniert werden. Beispiele solcher Gene sind das Dystrophin-Gen (Lee et al., Nature 349, 334, 1991, das Faktor VIII-Gen (Toole et al., Nature 312, 342 (?) 91984), das CFTR-Gen (Rommens et al., Science 245, 1059, 1989, Riordan et al., Science 245, 1066, 1989, das OTC-Gen (Horwich et al., Science 224, 1066, 1984, und das Alphal-Antitrypsin-Gen (Lemarchand et al., Proc. Natl. Acad. Sci. USA, 89,6482, 1992.

Beispiele für weitere Gene, die von Vektoren exprimiert werden, die in transformierten Amniozyten hergestellt werden können, sind das p53-Gen zur Behandlung von Tumorerkrankungen (Wills et al., Hum Gene Ther. 5, 1079, 1994, Clayman et al., Cancer Res. 55, 1, 1995, das Rb-Gen zur Behandlung von vaskulären proliferativen Erkrankungen (Chang et al., Science 267, 518, 1995, oder das Thymidin-Kinase-Gen von Herpes simplex-Virus (HSV) Typ I zur Therapie von Tumorerkrankungen. Das Gen, das von in transformierten Amniozyten hergestellten Vektoren exprimiert wird, muß nicht unbedingt für ein Protein kodieren. So können z.B. funktionelle RNAs exprimiert werden. Beispiele für solche RNAs sind Antisense-RNAs (Magrath, Ann. Oncol. 5, (Suppl 1), 67-70 1994, Milligan et al., Ann. NY Acad. Sci. 716, 228-241, 1994, Schreier, Pharma. Acta Helv., 68, 145-159 (1994), and katalytische RNAs (Cech, Biochem. Soc. Trans. 21, 229-234, 1993; Cech, Gene 135, 33-36, 1993; Long et al., FASEB J. 7, 25-30, 1993; Rosi et al., Pharm. Therap. 50, 245-254, 1991).

Vektoren, die in transformierten Amniozyten hergestellt werden, können zusätzlich zum therapeutischen Gen ein beliebiges Reporter-Gen enthalten, um die Expression des Vektors besser verfolgen zu können. Beispiele von Reporter-Genen sind im Stand der Technik bekannt und schließen z.B. das β-Galaktosidase-Gen ein (Fowler et al., Proc. Natl. Acad. Sci. USA 74, 1507, 1977).

Vektoren, die in transformierten Amniozyten hergestellt werden können, können mehr als ein einzelnes Gen enthalten. Die maximale Zahl an Genen, die in solchen Vektoren produzierten werden können, hängt von der Aufnahmekapazität des jeweiligen Vektors und von der Gengröße ab.

Die Wahl der Promoteren, die die Expression der therapeutischen Gene von in transformierten Amniozyten hergestellten Vektoren kontrollieren, ist nicht kritisch. Virale oder nicht-virale Promoteren, die konstitutiv, gewebespezifisch oder regulierbar aktiv sind, können für die Expression eines Proteins oder einer funktionellen RNA verwendet werden. Der SV40- oder Zytomegalie-Virus-Promoter (Andersson et al., J. Biol. Chem. 264, 8222-8229, 1964) kann z.B. zur konstitutiven Expression eines Gens verwendet werden. Die Verwendung des Muskelkreatin-Kinase (MCK)-Promoters erlaubt die gewebespezifische Expression eines Proteins oder einer funktionellen RNA in Skelett- und Herzmuskulatur. Die Genexpression kann quantitativ und qualitativ durch die Verwendung eines regulierbaren System kontrolliert werden (Furth et al., Proc. Natl. Acad. Sci. USA 91, 9302-9306, 1992).

In Vektoren, die in transformierten Amniozyten hergestellt werden können, können genetische Elemente eingeschlossen werden, die das Verhalten des Vektors innerhalb der Empfängerzelle beeinflussen. Solche Elemente sind z.B. Elemente, die das nukleäre Targeting der Vektor-DNA erleichtern (Hodgson, Biotechnology 13, 222-225, 1995).

Die Verwendung von derartig hergestellten Vektoren kann *in vitro* oder *in vivo* erfolgen. Ein *in vitro*-Gentransfer erfolgt außerhalb des Körpers z.B. durch Zufügen von Vektor zu Zellkulturzellen oder zu primären Zellen, die zum Zwecke des Gentransfers dem Körper entnommen worden sind. Beim *in vivo*-Gentransfer können Vektorpartikel in Abhängigkeit vom Gewebe, das transduziert werden soll, auf verschiedene Weise appliziert werden. Beispiele sind die Injektion in das arterielle oder venöse Gefäßsystem, die direkte Injektion in das betreffende Gewebe (z.B. Leber, Hirn, Muskel), die Instillation in das betreffende Organ (z.B. Lunge oder Gastrointestinaltrakt) oder die direkte Applikation auf eine Oberfläche (z.B. Haut oder Blase).

Die folgenden Figuren und Beispiel sollen die Erfindung näher erläutern, ohne sie darauf zu beschränken.

### BESCHREIBUNG DER FIGUREN

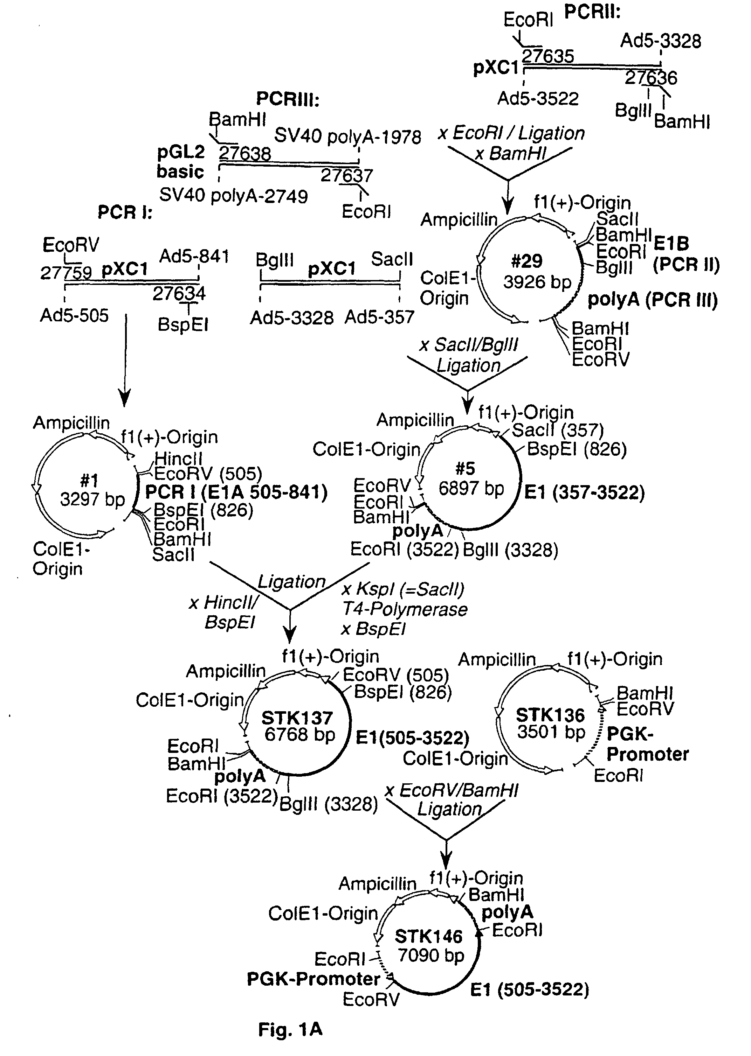

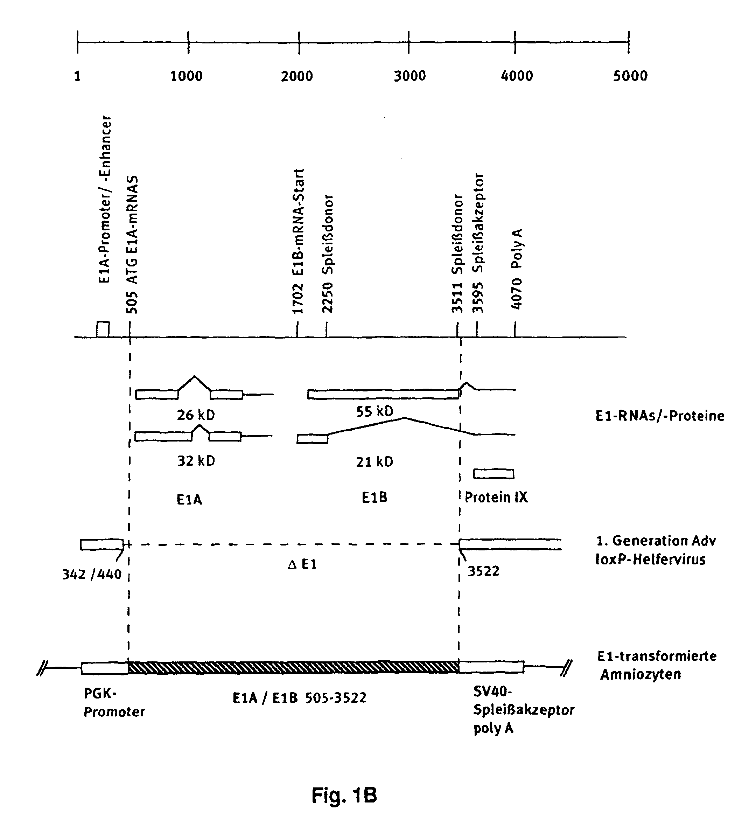

- Fig. 1: zeigt eine Übersicht über die Klonierungen: In Fig. 1A sind die Klonierungsschritte für Plasmid STK146 schematisch dargestellt. In Fig. 1B sind die linksterminalen ca. 15% des Genoms von Adenovirus Typ 5, einschließlich der E1-RNAs, der kodierenden Regionen, der Startpunkte der E1A- und E1B-Transkription, der für die Klonierung wichtigen Spleißdonor- und Spleißakzeptorstellen und Polyadenylierungssequenzen dargestellt. Wichtig ist, dass in der Klonierung für Plasmid STK146 die Spleißdonorstelle bei Basenpaar 3511 von Ad5 beibehalten wurde, die Spleißakzeptorstelle und das Polyadenylierungssignal jedoch durch entsprechende Funktionen von SV40 ersetzt wurden. Weiter wurde der E1A-Promoter von Ad5 durch den PGK-Promoter ersetzt. Somit enthält STK146 die Ad5-Sequenzen von Basenpaar 505-5322 und die mit diesem Plasmid transformierten Zelllinien enthalten keine Ad5-Sequenzen, die in adenoviralen Vektoren der ersten oder zweiten Generation oder in loxP-Helferviren enthalten sind.
- Fig. 2: zeigt die aus Amniozyten mittels Transformation durch adenoviralen E1-Funktionen erhaltenen Zellinseln (Fig. 2A und Fig. 2B), sowie die aus einzelnen Zellen klonierten Zelllinien N52.E6 (DSM ACC2416; Fig. 2C) und N52.F4 (Fig. 2D). Zu beachten ist, dass sich die Zelllinien bzw. Einzelzellklone morphologisch dadurch von den Amniozyten unterscheiden, dass sie in der Regel kleiner sind und in ihrer Vermehrung nicht kontaktinhibiert sind.
- Fig. 3: zeigt den Integrationsstatus von STK146 in acht verschiedenen E1-transformierten Amniozyten-Zelllinien mit Hilfe eines Southem Blots.
- Fig. 4: zeigt, aufgelistet in einer Tabelle, die Herstellung eines adenoviralen Vektors der ersten Generation in verschiedenen klonierten Zelllinien anhand von bfu (*blue forming units*) pro Zelle bzw. die Effizienz der Transfektion der entsprechenden Zelllinien anhand der Bildung von Plaques.
- Fig. 5: zeigt die Expression der E1A- und E1B-Proteine von Ad5 in elf klonierten Amniozyten-Zelllinien (Westem Blot).
- Fig. 6: zeigt den Zeitverlauf der Synthese rekombinanter adenoviraler Vektoren in zwei klonierten Zelllinien N52.E6 und N52.F4. Fig. 6A zeigt die Synthese eines adenoviralen Vektors der ersten Generation Fig. 6B die Synthese eines adenoviralen Vektors mit großer DNA-Kapazität.

### BEISPIELE

### 1. Klonierungen

Eine Übersicht über die Klonierungen ist in Fig. 1 dargestellt.

### a) Plasmid STK136

Plasmid STK136 enthält den murinen Phosphoglycerat-Kinase-Promoter (Seq. Nr. 1; Adra et al., Gene 60, 65-74, 1987) in pBluescript KSII (Stratagene) und wurde wie folgt hergestellt:

3.5 µg Plasmid PGK-hAAT (Kay et al., Hepatology 21, 815-819, 1995) wurden mit EcoRV verdaut und in einem 1.5%igen Agarosegel größenfraktioniert. Die 0.5 kb umfassende Bande, die das gesuchte PGK-Promoterfragment enthält, wurde nach Färbung in Ethidiumbromid ausgeschnitten und die DNA elektroeluiert. Zeitgleich wurde pBluescript KSII mit EcoRV und HincII verdaut und die freien DNA-Enden dephosphoryliert. Nach anschließender Phenol/Chloroform-Extraktion und Ethanolpräzipitation wurden äquimolare Mengen dieser DNA-Fragmente ligiert und in ultrakompetente XL-2 Blue-Bakterien (Stratagene) transformiert. Die Plasmid-Klone wurden mit Hilfe eines Restriktionsverdaus charakterisiert und das daraus resultierende Plasmid wurde mit STK136 (Isolat #6) bezeichnet.

### b) Plasmid STK137

Plasmid STK137 enthält die gesamte E1-Expressionskassette von Ad5 einschließlich des 3'-Spleiß- und Polyadenylierungssignals aus SV40 und wurde wie folgt hergestellt:

### PCR-Amplifikation der Ad5-Sequenz bp 505-841 (PCR I) (Seq. Nr. 2)

10 ng des Plasmids pXC1 (Microbix) wurden zusammen mit je 400 ng der Oligonukleotide 27759 (Seq. Nr. 3) und 27634 (Seq. Nr. 4), 0.2 mM dNTPs und 1.25 U Pfu-Polymerase in 10 mM KCl, 10 mM (NH₄)₂SO₄, 20 mM Tris/HCl, pH 8.75, 2 mM MgSO₄, 0.1% Triton X-100, 100µg/ml BSA unter folgenden Bedingungen amplifiziert:

| | |
|---|---|
| I | 10 Minuten bei 94°C, |
| II | 1 Minute bei 94°C, |
| | 2 Minuten bei 50°C, |
| | 3 Minuten bei 72°C, |
| III | 10 Minuten bei 72°C. |

wobei Schritt II in 15 Zyklen wiederholt wurde. Die DNA wurde mit Hilfe des "QIAquick PCR Purification Kit" (Qiagen) nach Angaben des Herstellers aufgereinigt und mit Ethanol präzipitiert.

Für die Klonierung des PCR-Fragmentes wurden 2.5 µg pBluescript KSII mit EcoRV vedaut, die freien DNA-Enden dephosphoryliert, in äquimolaren Mengen mit dem PCR-Fragment ligiert und in XL-2 Blue-Zellen transformiert. Das daraus resultierende Plasmid wird im nachfolgenden mit #1 bezeichnet.

### PCR-Amplifikation der Ad5 Sequenz bp 3328-3522 (PCR II) (Seq. Nr. 5):

10 ng des Plasmids pXC1 (Microbix) wurden zusammen mit je 400 ng der Oligonukleotide 27635 (Seq. Nr. 6) und 27636 (Seq. Nr. 7) unter den oben beschriebenen Bedingungen amplifiziert. Nach der PCR-Reaktion wurde die DNA mit Phenol/Chloroform extrahiert, mit Ethanol präzipitiert, mit EcoRI verdaut, nochmals mit Phenol/Chloroform extrahiert, präzipitiert und in 30µl TE gelöst.

### PCR-Amplifikation des 3'-Spleiß- und Polyadenylierungssignals aus SV40 mit Hilfe des Plasmids pGL2-Basic bp 1978-2749 (PCR III) (Seq. Nr. 8):

20 ng des Plasmids pGL2-Basic (Promega, GenBank/EMBL Acc. Nr.: X65323) wurden zusammen mit je 800 ng der Oligonukleotide 27637 (Seq. Nr. 9) und 27638 (Seq. Nr. 10), 0.4 mM dNTPs und 2.5 U Pfu-Polymerase unter den oben beschriebenen Bedingungen amplifiziert. Nach der PCR-Reaktion wurde die DNA mit Phenol/Chloroform extrahiert, mit Ethanol präzipitiert, mit EcoRI verdaut, nochmals mit Phenol/Chloroform extrahiert, in Ethanol präzipitiert und in 30 µl TE gelöst. Dann wurden je 10 µl DNA aus PCR II und III in einem Volumen von 50 µl ligiert, mit Phenol/Chloroform extrahiert, mit Ethanol präzipitiert und in einem Volumen von 100 µl mit BamHI verdaut. Nach erneuter Phenol/Chloroform-Extraktion und Ethanolpräzipitation wurde die DNA mit äquimolaren Mengen pBluescript KSII-DNA, welche zuvor mit BamHI verdaut und dephosphoyliert wurde, ligiert. Das daraus resultierende Plasmid wird nachfolgend mit #29 bezeichnet. Für die weitere Klonierung wurden 3.5 µg Plasmid-DNA #29 mit SacII und BglII verdaut, dephosphoryliert, mit Phenol/Chloroform extrahiert und in Ethanol präzipitiert. Zeitgleich wurden 3.5 µg pXC1 mit BglII und SacII verdaut, das 2.9 kb-Fragment elektrophoretisch aufgetrennt und elektroeluiert. Von beiden DNAs wurden äquimolare Mengen ligiert und in XL-2 Blue-Zellen transformiert. Das daraus resultierende Plasmid wird im nachfolgenden mit #5 bezeichnet. Für die abschließende Klonierung von STK137 wurde Plasmid #1 mit HincII und BspEI verdaut, elektrophoretisch aufgetrennt und ein ca. 350 bp umfassendes Fragment elektroeluiert. Als Vektor-DNA wurde Plasmid **#5** mit KspI (Isoschizomer von SacII) verdaut, die Enden mit T4-Polymerase aufgefüllt, mit Phenol/Chloroform extrahiert und mit Ethanol präzipitiert. Danach wurde die DNA mit BspEI verdaut, die Enden dephosphoyliert, nochmal mit Phenol/Chloroform extrahiert und mit Ethanol präzipitiert. Beide DNAs wurden ligiert und in XL-2 Blue-Zellen transformiert. Das daraus resultierende Plasmid wurde mit **STK137** (Isolat #34) bezeichnet.

### c) Plasmid STK146 (Seq. Nr. 18)

Plasmid STK146 enthält den murinen PGK-Promoter, die gesamte E1-Region von Ad5 (bp 505-3522) und das 3'-Spleiß- und Polyadenlylierungssignal von SV40.

Für die Klonierung wurden 4 µg STK137 mit EcoRV und BamHI verdaut, elektorphoretisch aufgetrennt und das 3.7 kb umfassende Fragment elektroeluiert. Weiter wurden 3.3 µg STK136 mit EcoRV und BamHI verdaut, dephosphoryliert, Phenol/Chloroform extrahiert und mit Ethanol präzipitiert. Von beiden Plasmiden wurden äquimolare Mengen ligiert und in XL-2 Blue-Zellen transformiert. Das daraus resultierende Plasmid wurde STK139 genannt. Eine abschließende Sequenzanalyse von STK139 ergab eine Mutation bei bp 2613 (die Nummerierung bezieht sich hierbei auf die Ad5-DNA-Sequenz), die zu einem Aminosäureaustausch von Tyrosin zu Asparagin führte (2613 TAC → GAC). Aus diesem Grunde wurde das die Mutation enthaltende Fragment in STK139 durch das BstEII (bp 1915)- BglII (bp 3328)-Fragment aus pXC1 ersetzt. Dazu wurde STK139 mit BstEII und BglII verdaut, dephosphoyliert, Phenol/Chloroform extrahiert, mit Ethanol präzipitiert, elektrophoretisch aufgetrennt und das 5.8 kb umfassende Fragment elektroeluiert. pXC1 wurde ebenfalls mit BstEII und BglII verdaut, elektrophoretisch aufgetrennt und das 1.4 kb umfassende Fragment elektroeluiert. Nach Ligation und Transformation wurde DNA aus 4 Plasmidklonen sequenziert; zwei davon enthielten die korrekte Sequenz bei bp 2613. Isolat Nummer 2 wurde komplett sequenziert und wird im nachfolgenden mit **STK146** bezeichnet.

### d) Sequenzanalyse von STK146

500 ng STK146 #2 wurden mit 10 pmol der folgenden Sequenzprimer unter Standardbedingungen sequenziert:

### 2. Kultivierung von primären Amniozyten und Zelllinien

Alle Zellkulturreagenzien, Medien und Seren wurden von der Firma GIBCO Life Technologies bezogen. Die Zelllinie 293, die in einigen Experimenten als Kontrolle diente, wurde in modifiziertem Eagles Medium (MEM) mit 10% fötalem Kälberserum (FCS), 1x Penicillin/Streptomycin bei 37°C (100x, Cat# 10378-016), 95% Luftfeuchtigkeit und 5% CO₂ kultiviert. Für die Herstellung der neuen E1-transformierten Zelllinien wurden primäre fötale Zellen, die im Rahmen der Pränataldiagnostik aus Fruchtwasser durch Amniozentese gewonnen worden waren, verwendet. Nach der Punktion wurden die Zellen, Routinemethoden folgend, in Plastikkulturflaschen ausgesäht und in Ham's F10-Medium (Nutrient Mixture Ham's F10 mit L-Glutamin, Cat# 31550-023), 10 % FCS, 2% Ultroser® G, 1x Antiobiotika/Antimykotika Lösung (100x, Cat# 15245-012), 2.5µg/ml Fungizione® (Amphotericin B, Cat# 15290-018), kultiviert. Ein Teil der Zellen wurde am Boden der Zellkulturflasche adhärent und proliferiert. Nach etwa 2 Wochen waren ausreichend Zellen für eine Chromosomenanalyse vorhanden. Nach Feststellung des Karyotyps wurden Amniozytenkulturen mit numerisch und strukturell unauffälligen Chromosomen für die Herstellung der Zelllinie verwendet. In verschiedenen Experimenten wurden Zellen von drei verschiedenen Quellen verwendet, deren Entnahme durch Amniozentese entweder 3, 6 bzw. 7 Wochen zurücklag. Als Nährmedium wurde Ham's F10-Medium, 10% FCS 2% Ultroser®Cr, 1x Antibiotika/Antimykotika-Lösung, 2.5 µg/ml Fungizione® verwendet. Die Kulturbedingungen waren 37°C, 95 % Luftfeuchtigkeit und 5% CO₂. Sieben Tage nach Transfektion wurden die Amniozyten in Ham's F-10-Medium, 10% FCS, 1x Penicillin/Streptomycin weiterkultiviert. Nach der Entstehung von Einzelzellklonen wurden diese auf neue Schalen übertragen und in alpha-MEM mit 10% FCS, 1x Penicillin/Streptomycin weiterkultiviert.

### 3. Transfektion und Transformation von Amniozyten

Für die Transfektion wurden die Amniozyten auf Zellkulturschalen (Durchmesser 60 mm, Oberfläche 22.1 cm²) mit einer Dichte von 2 - 5 x 10⁵ pro Schale ausgesät und am darauffolgenden Tag transfiziert. Für die Transfektion wurde 20 µg Plasmid STK146 mit ScaI verdaut, mit Phenol/Chloroform extrahiert, mit Ethanol präzipitiert und in 20 µl TE aufgenommen, was eine DNA-Konzentration von 0.5 µg/µl ergab. In ersten Experimenten wurden je 5 Schalen Ammniozyten 3 bzw. 7 Wochen nach Entnahme mit dem Effectene Transfektionskit nach Angaben des Herstellers (Qiagen) wie folgt transfiziert:

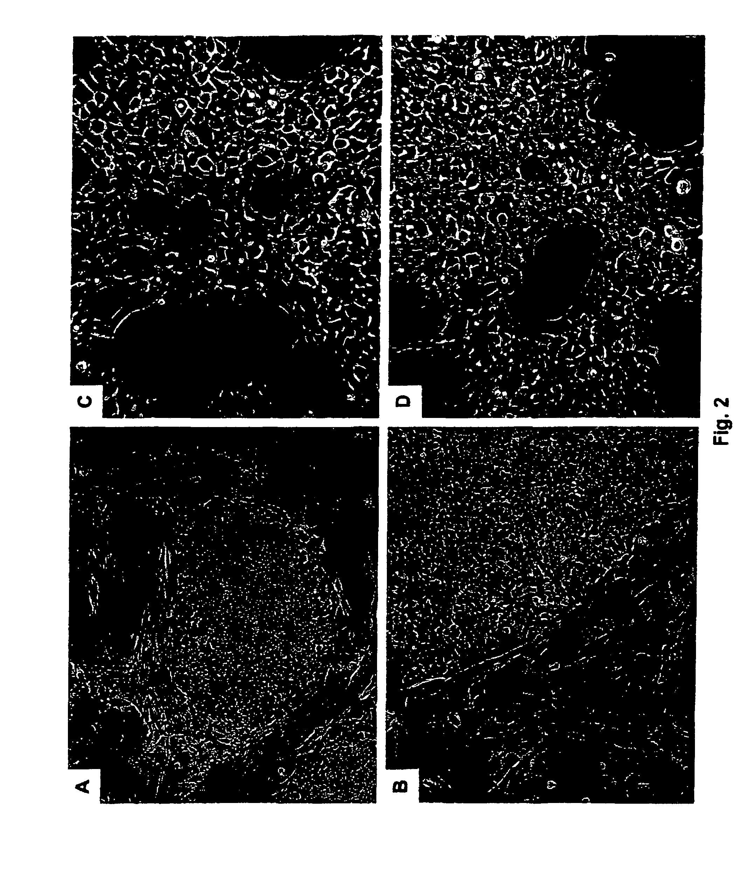

4 µl STK146, verdaut mit ScaI, wurden mit 146 µl EC Puffer gemischt. Nach Zugabe von 8 µl Enhancer wurde die Lösung kurz gevortext und 5 Minuten bei Raumtemperatur inkubiert. Danach wurden 25 µl Effectene zugegeben, 10 Sekunden gevortext und weitere 10 Minuten bei Raumtemperatur inkubiert. Währenddessen wurde das Medium von den Zellen vorsichtig abgesaugt und durch 4 ml frisches Nährmedium (siehe oben Punkt 2.) ersetzt. Nach abgeschlossener Inkubation wurde der Transfektionsansatz mit 1 ml frischem Nährmedium gemischt und vorsichtig auf die Zellen getropft. Die Zellen wurden weiter wie oben beschrieben kultiviert. Sieben Tage nach der Transfektion wurden die Zellen je einer Schale auf eine größere Schale (Durchmesser 150 mm, Oberfläche 147.8 cm²) übertragen. Dazu wurde das Medium vorsichtig abgesaugt, die Zellen mit PBS gewaschen, in Trypsin abgelöst und auf eine neue Schale übertragen und wie unter Punkt 2. beschrieben weiterkultiviert. 18 bis 22 Tage nach der Transfektion waren deutlich klonale Zellinseln zu beobachten, die sich morphologisch deutlich von den Amnionzellen unterschieden (Abb. 2A, 2B). Der Hauptanteil in einer nicht-transformierten Amniozytenkultur besteht aus größeren Zellen, die in ihrem Wachstum Kontaktinhibition zeigen. Zellen in transformierten Einzelzellkolonien sind sehr viel kleiner, wachsen viel schneller und sind in ihrer Vermehrung nicht kontaktinhibiert. Sie wachsen als Zellinseln, bestehend aus dicht gedrängten, kleineren Zellen und sind lichtmikroskopisch eindeutig und ohne Schwierigkeiten zu identifizieren. Diese Zellinseln wurden gepickt und auf eine neue Schale (Durchmesser 60 mm) mit oben beschriebenem Medium übertragen. Nach weiterer Vermehrung wurden die Zelllinien auf 147.8 cm² Zellkulturschalen übertragen und wie unter 2. beschrieben weiterkultiviert. Nach der ersten Übertragung auf 147.8 cm² Zellkulturschalen wurden die Passagen der Zellen gezählt. Anfänglich wurden ca. 40 Zellklone von den Zellen, die 3 bzw. 7 Wochen nach Entnahme transfiziert worden waren, weiterkultiviert. Im weiteren Verlauf, d.h. durch längeres Kultivieren, änderten einige der Zellklone ihre Morphologie drastisch und verhielten sich in ihrem Wachstum instabil. Die weiteren Experimente wurden auf die Weiterkultivierung und Analyse von acht morphologisch stabilen Zelllinien beschränkt. Diese wurden wie folgt bezeichnet: GS.A55 (enstanden aus Amniozyten transfiziert 3 Wochen nach Entnahme), GS.N21, GS.N24, GS.N27, GS.N49, GS.N51, GS.N52, GS.N53 (entstanden aus Amniozyten transfiziert sieben Wochen nach Entnahme).

Während der ersten Passagen zeigten alle Zellklone eine vergleichbare Morphologie, die sich durch weiteres Passagieren jedoch veränderte. So veränderten sich z. B. einige Zellklone dahin, daß sie eine stark abgerundete Form annahmen und nach weiteren Passagen nicht mehr adhärent waren. Andere Zellklone zeigten eine starke Vakuolisierung, die jedoch keinen Einfluß auf ihr Wachstum zu haben schien. Nach der Einzelzellklonierung zeigten alle Zelllinien eine einheitliche Morphologie und z.B. N52. EG und N52. F4 zeigten epitheliales Aussehen. Sie waren in ihrer Wachstumsrate und Zelldichte mit 293-Zellen zu vergleichen.

### 4. Effizienz der Transformation

Um die Effizienz der Transformation durch die E1-Funktionen genauer zu bestimmen, wurden in einem weiteren Ansatz erneut sieben Schalen mit je 2-5 x 10⁵ Zellen wie unter Punkt 3. beschrieben transfiziert. Bereits 24 Stunden nach Transfektion wurden die Zellen auf 147.8 cm² große Schalen übertragen und für 5 Tage in Ham's F-10-Medium, 10 % fötales Kälberserum, 2% Ultroser® G, 1 x Antiobiotika/Antimyotika Lösung, 2.5µg/ml Fungizione und weitere 25 Tage in Ham's Medium, 10 % fötales Kälberserum, 1 x Penicillin/Streptomycin-Lösung weiterkultiviert. Als Kontrolle wurde eine Schale mit nichttransfizierten Zellen unter denselben Bedingungen kultiviert. Während dieser Zeit wurden die morphologisch deutlich unterscheidbaren (siehe Fig. 2A, B) Kolonien, die aus einzelnen Transformationserreignissen entstanden waren, ausgezählt. Auf allen Zellkulturschalen mit Ausnahme der untransfizierten Kontrollschale konnten Einzelzellklone gezählt werden. Im Durchschnitt ergaben sich 4 Zellklone pro Platte, was einer Transformationseffizienz von 1 in 0.5-1 x 10⁵ Zellen entsprach.

### 5. Einzelzellklonierung

Wie bereits erwähnt verhielten sich die Zelllinien morphologisch teilweise unterschiedlich, weshalb von jeder Zelllinie bis zu zehn Einzel-Zelllinien angelegt wurden. Dabei befanden sich die einzelnen Zelllinien in verschiedenene Passagen: GS.A55: P17, GS.N21: P24, GS.N24: P20, GS.N27: P19, GS.N49: P21, GS.N51: P39, GS.N52: P22, GS.N53: P20. Dazu wurden die Zellen von den Zellkulturschalen abgelöst und bei einer Konzentration von 5 x 10⁶ Zellen/ml 1:1000, 1:50.000 und 1:500.000 in Nährmedium verdünnt. Von allen Verdünnungen wurden 100 µl Zellen auf 96 well-Platten ausgesät und diejenigen Zellklone, die eindeutig aus Einzelzellen entstanden waren weiterkultiviert. In Fig. 2C ist die Zelllinie GS.N52.E6 (DMSZ-Nr.) gezeigt, Fig. 2D zeigt Zelllinie GS.N52.F4; beide Zellklone stammen von der ursprünglichen Zelllinie GS.N52 ab.

### 6. Charakterisierung der E1-Zelllinien

### a) Southern Blot-Analysen

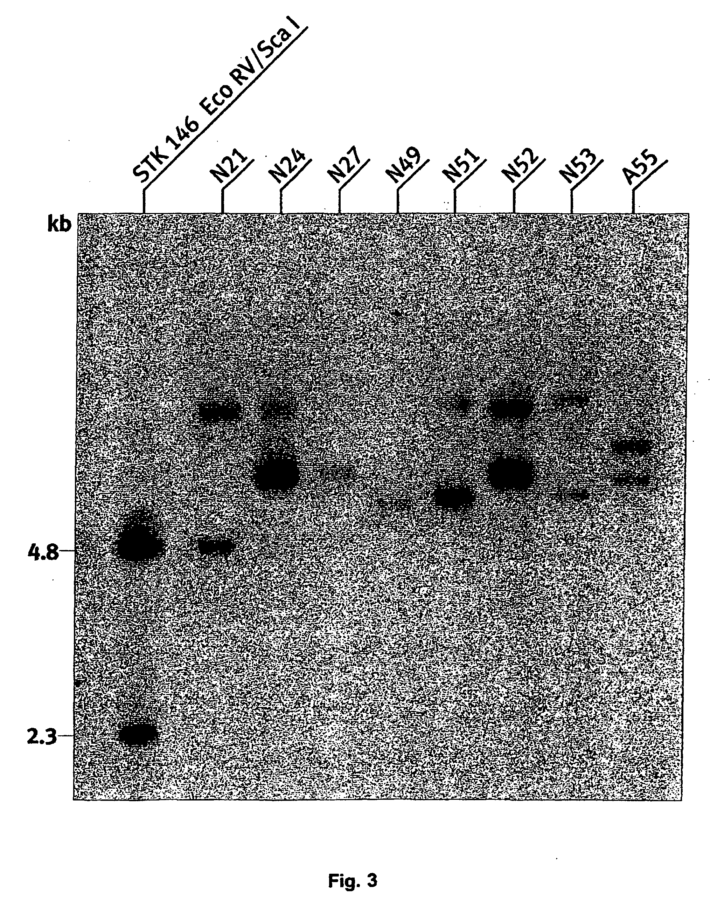

Um den Integrationsstatus der E1-Region in den Zelllinien zu untersuchen, wurden Southem Blot-Analysen durchgeführt. Dazu wurde genomische DNA aus allen acht E1-Amnionklonen (siehe Punkt 5.) isoliert, je 5 µg wurden mit EcoRV verdaut, elektorphoretisch aufgetrennt und auf eine Nylonmembran übertragen. EcoRV schneidet einmal in der E1-Expressionskassette. Die Hybridisierung mit radioaktiv markierter STK146-DNA bestätigte die Integration von 1-2 Kopien STK146 in allen Klonen. Fig. 3 zeigt das Integrationsmuster in den E1-Zellklonen. Bei allen Klonen sind hauptsächlich zwei hochmolekulare Banden zu erkennen, was auf die Integration einer einzelnen Kopie hindeutet. Die Zellklone GS.N24 und GS.N52 zeigten zusätzliche je eine Bände mit stärkerer Intensität, was auf die Integration von Tandem-Kopien von STK146 hindeuten könnte. Bei keinem der Zellklone traten Banden auf, die kleiner als STK146, verdaut mit ScaI und EcoRV waren, was darauf schließen liess, dass alle Integrate vollständig und nicht deletiert vorlagen.

### b) Erzeugung rekombinanter Adenoviren

Nach der Einzelzellklonierung wurden die Zellklone im Hinblick auf ihr Vermögen, rekombinantes Adenovirus zu erzeugen, getestet. Dazu wurden einmal 3-5 Einzelzellklone jeder Zelllinie in ca. 70% konfluenten 24 well-Platten mit ca. 5 moi (*mulliplicity of infection*) Adßgal (rekombinanter adenoviraler Vektor der ersten Generation) infiziert. 48 Stunden nach Infektion wurden die Zellen durch dreimaliges Einfrieren und Auftauen lysiert und die Menge an entstandenem Adßgal durch Infektion von 293-Zellen und anschließendes Färben der Zellen (MacGregor et al., in: Gene Transfer and Expression Protocolls, Murray Hrsg., Humana, Clifton, NJ, Bd. 7, S. 217-235, Jahr (1991)) zum Nachweis der β-Galaktosidase-Herstellung analysiert (Fig. 4). Diese Methode ergibt eine nur ungefähre Herstellung an rekombinantem Adenovirus, da sich die Zellzahlen und daher die verwendete Virusmenge bei den unterschiedlichen Zellklonen aufgrund ihrer Größe und Wachstumsgeschwindigkeit unterscheiden können. Die Zellklone, die in diesem ersten Test die größte Ausbeute ergaben, wurden in einem weiteren Experiment genauer analysiert. Dazu wurden auf 3 Schalen (Durchmesser 100 mm, Oberfläche 60 cm²) ca. 3 x 10⁷ Zellen ausgesät. Am darauffolgenden Tag wurden die Zellen ausgezählt und anhand der ermittelten Zellzahl mit genau 5 moi Adβgal infiziert. 48 Stunden nach Infektion wurden die Zellen geerntet, durch dreimaliges Einfrieren und Auftauen lysiert und die erzeugte Menge an Adβgal durch Infektion von 293-Zellen und anschließendes Färben analysiert. Das Ergebnis ist in Fig. 4 dargestellt.

### c) Transfektionseffizienz

Einige der klonierten Zelllinien wurden auf die Bildung von Plaques getestet. Dazu wurden ca. 70% konfluente Zellkulturschalen (Durchmesser 60 mm) mit 2 µg infektiösem Plasmid GS66 mit Hilfe der Kalciumphosphat-Methode transfiziert. Plasmid GS66 enthält das gesamte Adenovirusgenom mit einer Deletion in der E1-Region von Nukleotid 440 bis zu Nukleotid 3523. Die adenoviralen terminalen Wiederholungssequenzen (ITRs) sind in diesem Plasmid flankiert von SwaI-Restriktionsschnittstellen, so dass nach Transfektion von SwaI-verdautem Plasmid infektiöses Virus und Plaques entstehen können. Ca. 24 Stunden nach Transfektion wurden die Zellen mit ca. 10 ml MEM, 1% Agarose, 0.5x Penicillin/Strepromycin, 0.05% Hefeextrakt überschichtet. Nach ca. 1 Woche Inkubation bei 37°C, 95% Luftfeuchtigkeit, 5% CO₂ wurden Plaques sichtbar. Fig. 4 zeigt die Anzahl ausgezählter Plaques, gemittelt aus je 2 unabhängigen Transfektionen.

### d) Expression der E1A- und E1B-Funktionen von Ad5

Die Expression der Ad5-E1A- und der E1B-21kD-Proteine in den klonierten Zelllinien wurde mit Hilfe von Western Blot-Analysen unter Verwendung von monoklonalen Antikörpern nachgewiesen.

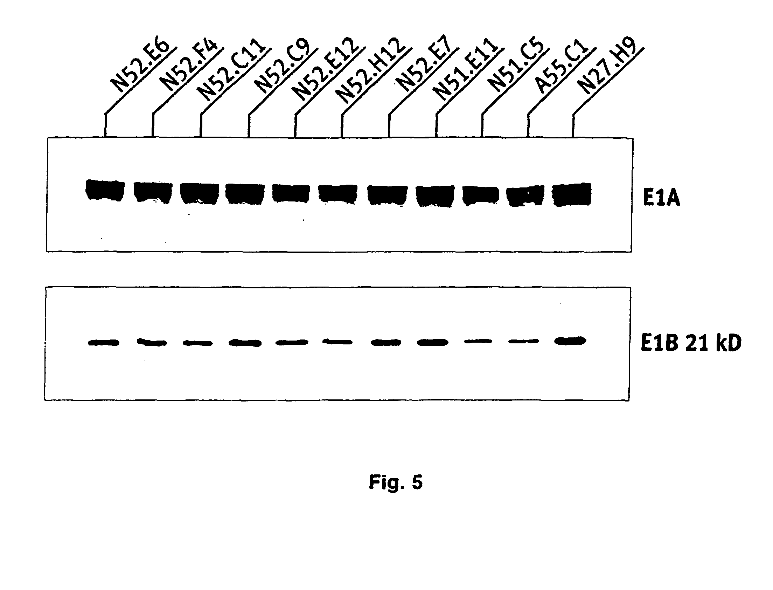

Von einer Zellkulturschale (Durchmesser 10 cm) wurden die Zellen in PBS/1mM EDTA vorsichtig abgelöst, pelletiert und in 150 µl 50 mM Tris/HCL pH 8, 140 mM NaCl, 0.5% NP40, 4 mM EDTA, 2 mM EGTA, 0.5 mM PMSF, 5% Glycerin aufgenommen. Durch zusätzliches "Douncen" wurden die Zellen lysiert, 10 Minuten bei 13 000 Upm abzentrifugiert und die Proteinkonzentration im Überstand mit Hilfe eines Proteinbestimmungskits (BIORAD, Microassay Procedure) bestimmt. Auf einem 12%-igen SDS-Polyacrylamidgel wurden 10 µg Protein aufgetrennt, auf eine Nitrozellulosemembran (Hybond ECL, Amersham Pharmacia Biotech) transferiert und mit einem anti-E1A- bzw. anti-E1B-21kD-Antikörper (Calbiochem, Verdünnung 1:300) inkubiert. Am darauffolgenden Tag wurde der Blot gewaschen und mit einem zweiten anti-Maus-Antikörper (EIA) bzw. anti-Ratte-Antikörper (E1B), an den Meerrettichperoxidase gekoppelt war, hybridisiert. Die Reaktion der Meerettichperoxidase wurde durch Inkubation gleicher Volumina an "Enhance-Lösung" 1 und 2 (ECL, Amersham Pharmacia Biotech) gestartet und die sich dadurch entwickelnde photochemische Reaktion durch kurze Exposition eines Röntgenfilms sichtbar gemacht. Fig. 5 zeigt das Ergebnis einer Western Blot-Analyse.

### e) Zeitverlauf der Synthese rekombinanter adenoviraler Vektoren der ersten Generation und adenoviraler Vektoren mit großer DNA-Kapazität

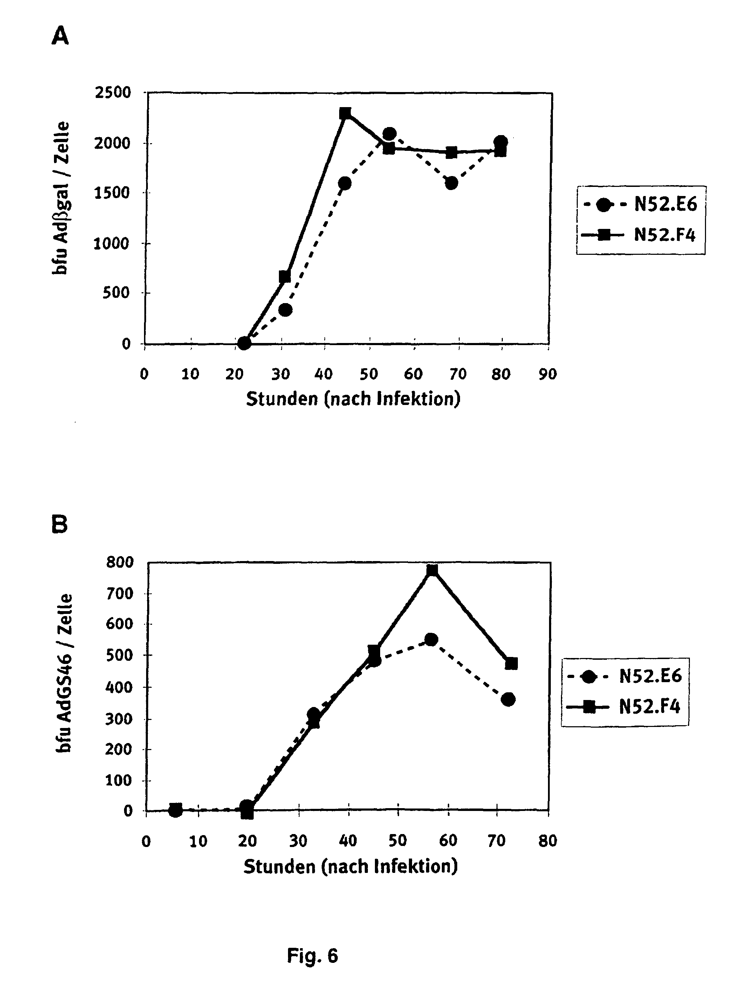

Zwei klonierte Zelllinien, N52.E6 und N52.F4, zeigten in den oben beschriebenen Experimenten die höchste Ausbeute an rekombinanten adenoviralen Vektoren der ersten Generation. Für die optimale Herstellung von adenoviralen Vektoren sind weitere Kenntnisse über den Zeitverlauf wichtig, insbesondere dann, wenn diese Zellen auf Suspensionskulturen adaptiert werden und eine erfolgreiche Infektion nicht anhand eines zytopathischen Effektes verfolgt werden kann. Für diese Analyse wurden mehrere Schalen (Durchmesser 6 cm) mit je 3 x 10⁶ Zellen mit 5 moi Adβgal infiziert und zu den angegebenen Zeitpunkten nach Infektion geerntet. Die Ausbeute an rekombinatem Adenovirus wurde wieder durch Infektion von 293-Zellen, Färbung und Auszählen blauer Zellen ermittelt (Fig. 6 A).

Zukünftig ist vorgesehen, die neuen Zelllinien auch für die Herstellung adenoviraler Vektoren mit großer DNA-Kapazität zu verwenden (siehe oben). Für die Herstellung dieser adenoviralen Vektoren werden Helferviren benötigt, die die deletierten Funktionen und Proteine für einen lytischen Infektionszyklus in *trans* zur Verfügung stellen. Das Verpackungssignal dieser Helferviren wird mit Hilfe von loxP-Erkennungssequenzen und der Cre-Rekombinase, welche von der Zelllinie exprimiert wird, bei Infektion entfernt. In zukünftigen Experimenten sollen daher die neuen E1-transformierten Zelllinien mit einem Cre-exprimierenden Plasmid transfiziert und die Rekombinase stabil exprimiert werden.

In vorläufigen Experimenten wurde getestet, ob die neuen E1-Amniozyten auch in der Lage sind, adenovirale Vektoren mit großer DNA-Kapazität zu erzeugen und ob die Produktionskinetik und die Menge an produzierten Vektoren der entspricht, die mit bereits existierenden Cre-exprimierenden 293-Zellen erreicht wird. Dazu wurden mehrere Schalen mit je 3 x 10⁶ Zellen der Zelllinien N52.E6 und N52.F4 mit 5 moi loxP-Helfervirus und 10 moi AdGS46 (β-gal exprimierender adenoviraler Vektor mit großer DNA-Kapazität) infiziert und zu den angegebenen Zeitpunkten nach Infektion geerntet. Die Ausbeute an β-gal exprimierendem adenoviralen Vektor mit großer DNA-Kapazität wurde wieder durch Infektion von 293-Zellen, Färbung und Auszählen blauer Zellen ermittelt (Fig. 6B). Die Menge der in den Amniozyten synthetisierten adenoviralen Vektoren mit großer DNA-Kapazität entspricht der, die auch in Cre-experimierenden 293-Zellen erzeugt wird (Daten nicht gezeigt).

## Patentansprüche

1. Permanente Amniozyten-Zelllinie, enthaltend mindestens eine Nukleinsäure, die die Expression der Genprodukte der E1A- und E1B-Region von Adenovirus bewirkt.

2. Zelllinie nach Anspruch 1, wobei die mindestens eine Nukleinsäure weiterhin die Expression der Genprodukte der E2A-, E2B- und/oder E4-Region von Adenovirus und/oder der Cre-Rekombinase bewirkt.

3. Zelllinie nach Anspruch 1 oder 2, wobei die Expression des Genprodukts der E1A-Region unter der Kontrolle eines konstitutiven Promoters, vorzugsweise des Phosphoglycerat-Kinase(PGK)-Promoters, steht.

4. Zelllinie nach einem der Ansprüche 1 - 3, wobei die Expression des/der Genprodukte der E1B-Region unter der Kontrolle eines adenoviralen Promoters, vorzugsweise des adenoviralen E1B-Promoters, steht.

5. Zelllinie nach einem der Ansprüche 1 - 4, wobei die adenoviralen Genprodukte vom humanen Adenovirus Typ 5 stammen.

6. Zelllinie nach einem der Ansprüche 1 - 5, wobei die Zelllinie eine humane Zelllinie ist.

7. Verfahren zur Herstellung einer permanenten Amniozyten-Zelllinie, **gekennzeichnet durch** das Transfizieren von Amniozyten mit mindestens einer Nukleinsäure, die die Expression der adenoviralen Genprodukte der E1A-Region und E 1B-Region bewirkt.

8. Verfahren nach Anspruch 7, wobei primäre Amniozyten, insbesondere humane primäre Amniozyten, verwendet werden.

9. Verfahren nach Anspruch 7 oder 8, wobei die genannte Nukleinsäure in Form eines Expressionsvektors verwendet wird.

10. Verfahren nach einem der Ansprüche 7 - 9, wobei das Genprodukt der E1A-Region unter der Kontrolle eines konstitutiven Promoters, vorzugsweise des Phosphoglycerat-Kinase(PGK)-Promoters, und das/die Genprodukte der E1B-Region unter der Kontrolle eines adenoviralen Promoters, vorzugsweise des adenoviralen E1B-Promoters, exprimiert werden.

11. Verfahren nach einem der Ansprüche 7 - 10, wobei durch Transfizieren der Amniozyten und/oder der erhaltenen Zelllinie zusätzlich die Expression der Genprodukte der E2A- und/oder E2B- und/oder E4-Region von Adenovirus und/oder der Cre-Rekombinase bewirkt wird.

12. Verfahren nach einem der Ansprüche 7 - 11, wobei die adenoviralen Genprodukte vom humanen Adenovirus Typ 5 stammen.

13. Permanente Amniozyten-Zelllinie erhältlich gemäß dem Verfahren nach einem der Ansprüche 7 - 12.

14. Permanente Amniozyten-Zelllinie N52.E6 (DSM ACC2416).

15. Verwendung von Amniozyten zur Herstellung von Adenovirus-transformierten permanenten Amniozyten-Zelllinien.

16. Verwendung der adenoviralen Genprodukte der E1A- und E1B-Region zur Herstellung von permanenten Amniozyten-Zelllinien.

17. Verwendung einer permanenten Amniozyten-Zelllinie zur Herstellung von Gentransfervektoren und/oder Adenovirusmutanten.

18. Verwendung nach Anspruch 17 zur Herstellung von Adenovirusvektoren, AAV(Adeno-Assoziiertes Virus)-Vektoren, Retrovirusvektoren, Lentivirusvektoren, chimären Adenovirus-AAV-Vektoren, chimären Adenovirus-Retrovirus-Vektoren und/oder chimären Adenovirus-Lentivirus-Vektoren.

19. Verwendung nach Anspruch 18, wobei die Adenovirusvektoren Adenovirusvektoren der ersten Generation, Adenovirusvektoren der zweiten Generation, Adenovirusvektoren großer DNA-Kapazität und/oder deletierte Adenovirusvektoren sind.

20. Verwendung nach einem der Ansprüche 17 - 19 zur Herstellung von Tropismus-modifizierten Gentransfervektoren und/oder Tropismus-modifizierten Adenovirusmutanten.

21. Verwendung nach einem der Ansprüche 17 - 20, wobei die Amniozyten-Zelllinie wie in einem der Ansprüche 1 - 6, 13 oder 14 definiert ist.

22. Verfahren zur Herstellung von Gentransfervektoren und/oder Adenovirusmutanten, wobei eine permanente Amniozyten-Zelllinie verwendet wird.

## Claims

1. A permanent amniocytic cell line comprising at least one nucleic acid which brings about expression of the gene products of the adenovirus E1A and E1B regions.

2. The cell line of claim 1, wherein the at least one nucleic acid also brings about theexpression of the gene products of the adenovirus E2A, E2B and/or E4 regions and/or of Cre recombinase.

3. The cell line of claim 1 or 2, wherein the expression of the gene product of the E1A region is under the control of a constitutive promoter, preferably of the phosphoglycerate kinase (PGK) promoter.

4. The cell line of any of claims 1-3, wherein expression of the gene product(s) of the E1B region is under the control of an adenoviral promoter, preferably of the adenoviral E1B promoter.

5. The cell line of any of claims 1-4, wherein the adenoviral gene products are derived from human adenovirus type 5.

6. The cell line of any of claims 1-5, wherein the cell line is a human cell line.

7. A process for producing a permanent amniocytic cell line, **characterized by** the transfection of amniocytes with at least one nucleic acid, which brings about the expression of the adenoviral gene products of the E1A region and E1B region.

8. The process of claim 7, wherein primary amniocytes, in particular human primary amniocytes, are used.

9. The process of claim 7 or 8, wherein said nucleic acid is used in the form of an expression vector.

10. The process of any of claims 7 - 9, wherein the gene product of the E1A region is expressed under the control of a constitutive promoter, preferably of the phosphoglycerate kinase (PGK) promoter, and the gene product(s) of the E1B region is expressed under the control of an adenoviral promoter, preferably of the adenoviral E1B promoter.

11. The process of any of claims 7-10, wherein the transfection of the amniocytes and/or of the resulting cell line additionally brings about the expression of the gene products of the adenovirus E2A and/or E2B and/or E4 regions and/or of Cre recombinase.

12. The process of any of claims 7 - 11, wherein the adenoviral gene products are derived from human adenovirus type 5.

13. A permanent amniocytic cell line obtainable by the process of any of claims 7 - 12.

14. The permanent amniocytic cell line N52.E6 (DSM ACC2416).

15. Use of amniocytes for producing adenovirus-transformed permanent amniocytic cell lines.

16. Use of the adenoviral gene products of the E1A and E1B regions for producing permanent amniocytic cell lines.

17. Use of a permanent amniocytic cell line for producing gene transfer vectors and/or adenovirus mutants.

18. The use according to claim 17 for producing adenovirus vectors, AAV (adeno-associated virus) vectors, retrovirus vectors, lentivirus vectors, chimeric adenovirus-AAV vectors, chimeric adenovirus-retrovirus vectors and/or chimeric adenovirus-lentivirus vectors.

19. The use according to claim 18, wherein the adenovirus vectors are first-generation adenovirus vectors, second-generation adenovirus vectors, adenovirus vectors of large DNA capacity and/or deleted adenovirus vectors.

20. The use according to any of claims 17-19 for producing tropism-modified gene transfer vectors and/or tropism-modified adenovirus mutants.

21. The use according to any of claims 17-20, wherein the amniocytic cell line is as defined in any of claims 1-6, 13 or 14.

22. A process for producing gene transfer vectors and/or adenovirus mutants, wherein a permanent amniocytic cell line is used.

## Revendications

1. Lignée cellulaire permanente d'amniocytes, comprenant au moins un acide nucléique, qui dirige l'expression des produits des gènes de la région E1A et E1B d'un adénovirus.

2. Lignée cellulaire selon la revendication 1, dans laquelle au moins un acide nucléique dirige en outre l'expression des produits des gènes de la région E2A, E2B et E4 d'un adénovirus et/ou de la recombinase Cre.

3. Lignée cellulaire selon la revendication 1 ou la revendication 2 dans laquelle l'expression du produit du gène de la région E1A est placée sous le contrôle d'un promoteur constitutif, de préférence le promoteur de la phosphoglycérate-kinase (PGK).

4. Lignée cellulaire selon l'une des revendications 1 à 3, dans laquelle l'expression du/des produit(s) du (des) gène(s) de la région E1B est placée sous le contrôle d'un promoteur adénoviral, de préférence le promoteur adénoviral E1B.

5. Lignée cellulaire selon l'une des revendications 1 à 4, dans laquelle les produits des gènes adénoviraux sont dérivés de l'adénovirus de type 5 humain.

6. Lignée cellulaire selon l'une des revendications 1 à 5, dans laquelle la lignée cellulaire permanente est une lignée cellulaire humaine.

7. Procédé de fabrication d'une lignée cellulaire d'amniocytes humains, **caractérisé par** la transfection d'amniocytes avec au moins un acide nucléique qui dirige l'expression des produits des gènes adénoviraux de la région E1A et de la région E1B.

8. Procédé selon la revendication 7, dans lequel sont utilisés des amniocytes primaires, en particulier des amniocytes primaires humains.

9. Procédé selon la revendication 7 ou la revendication 8, dans lequel lesdits acides nucléiques sont utilisé sous forme d'un vecteur d'expression.

10. Procédé selon l'une des revendications 7 à 9, dans lequel le produit du gène de la région E1A est exprimé sous le contrôle d'un promoteur constitutif, de préférence le promoteur de la phosphoglycérate-kinase (PGK), et le ou les produit(s) du ou des gène(s) de la région E1B est (sont) exprimé(s) sous le contrôle d'un promoteur adénoviral, de préférence, le promoteur adénoviral E1B.

11. Procédé selon l'une des revendications 7 à 10, dans lequel l'expression des produits des gènes de la région E2A et/ou E2B et/ou E4 d'un adénovirus et/ou de la recombinase Cr est en outre dirigée par transfection des amniocytes et/ou de la lignée cellulaire obtenue.

12. Procédé selon l'une des revendications 7 à 11, dans lequel les produits des gènes adénoviraux sont dérivés de l'adénovirus de type 5 humain.

13. Lignée cellulaire permanente d'amniocytes pouvant être obtenue selon le procédé de l'une des revendications 7 à 12.

14. Lignée cellulaire permanente d'amniocytes N° 52.E6 (DSM ACC 2416).

15. Utilisation d'amniocytes pour la fabrication de lignées cellulaires permanentes d'amniocytes transformées par un adénovirus.

16. Utilisation des produits des gènes adénoviraux de la région E1A et E1B pour la fabrication d'une lignée cellulaire permanente d'amniocytes.

17. Utilisation d'une lignée cellulaire permanente d'amniocytes pour la fabrication d'un vecteur de transfert de gène et/ou d'un mutant d'un adénovirus.

18. Utilisation selon la revendication 17 pour la fabrication de vecteurs adénoviraux, vecteurs AAV (virus adéno-associé), vecteurs rétroviraux, vecteurs lentiviraux, vecteurs chimères adénovirus-AAV, vecteurs chimères adénovirus - rétrovirus et/ou vecteurs chimères adénovirus-lentivirus.

19. Utilisation selon la revendication 18, dans laquelle les vecteurs adénoviraux sont des vecteurs adénoviraux de première génération, des vecteurs adénoviraux de deuxième génération, des vecteurs adénoviraux à grande capacité d'ADN et/ou des vecteurs adénoviraux délétés.

20. Utilisation selon l'une des revendications 17 à 19 pour la fabrication de vecteurs de transfert de gène à tropisme modifié et/ou de mutants adénoviraux à tropisme modifé.

21. Utilisation selon l'une des revendications 17 à 20, dans laquelle la lignée cellulaire d'amniocytes est définie conformément à l'une des revendications 1 à 6, 13 ou 14.

22. Procédé de fabrication de vecteurs de transfert de gènes et/ou de mutants adénovirus, dans lequel on utilise une lignée permanente d'amniocytes.


### SEQUENZPROTOKOLL

<110> Kochanek, S.
<120> Permanente Amniozyten-Zelllinie, ihre Herstellung und Verwendung zur Herstellung von Gentransfervektoren
<160> 18
<170> Word 6.0, PC-DOS/MS-DOS
<210> 1
   <211> 513
   <212> DNA
   <213> Mus sp.
   <223> Phosphoglycerat-Kinase-Promoter
<400> 1
   1    gaattctacc gggtagggga ggcgcttttc ccaaggcagt ctggagcatg
   51   cgctttagca gccccgctgg cacttggcgc tacacaagtg gcctctggcc
   101  tcgcacacat tccacatcca ccggtaggcg ccaaccggct ccgttctttg
   151  gtggcccctt cgcgccacct tctactcctc ccctagtcag gaagttcccc
   201  cccgccccgc agctcgcgtc gtgcaggacg tgacaaatgg aagtagcacg
   251  tctcactagt ctcgtgcaga tggacagcac cgctgagcaa tggaagcggg
   301  taggcctttg gggcagcggc caatagcagc tttgctcctt cgctttctgg
   351  gctcagaggc tgggaagggg tgggtccggg ggcgggctca ggggcgggct
   401  caggggcggg gcgggcgccc gaaggtcctc cggaggcccg gcattctcgc
   451  acgcttcaaa agcgcacgtc tgccgcgctg ttctcctctt cctcatctcc
   501  gggcctttcg acc
<210> 2
   <211> 337
   <212> DNA
   <213> Humanes Adenovirus Typ 5
   <223> Basenpaare 505-841
<400> 2
   1    gagtgccagc gagtagagtt ttctcctccg agccgctccg acaccgggac
   51   tgaaaatgag acatattatc tgccacggag gtgttattac cgaagaaatg
   101  gccgccagtc ttttggacca gctgatcgaa gaggtactgg ctgataatct
   151  tccacctcct agccattttg aaccacctac ccttcacgaa ctgtatgatt
   201  tagacgtgac ggcccccgaa gatcccaacg aggaggcggt ttcgcagatt
   251   tttcccgact ctgtaatgtt ggcggtgcag gaagggattg acttactcac
   301  ttttccgccg gcgcccggtt ctccggagcc gcctcac
<210> 3
   <211> 29
   <212> DNA
   <213> künstliche Sequenz
   <220>
   <223> Oligonukleotid 27759 zur PCR-Amplifikation der Sequenz bp 505 - 841 des humanen Adenovirus Typ 5
<400> 3
   atcgagtgcc agcgagtaga gttttctcc 29
<210> 4
   <211> 22
   <212> DNA
   <213> künstliche Sequenz
   <220>
   <223> Oligonukleotid 27634 zur PCR-Amplifikation der Sequenz bp 505 - 841 des humanen Adenovirus Typ 5
<400> 4
   gtgaggcggc tccggagaac cg 22
<210> 5
   <211> 216
   <212> DNA
   <213> Humanes Adenovirus Typ 5
   <223> Basenpaare 3328-3522
<400> 5
   1 ctcgcggatc cagatctgga aggtgctgag gtacgatgag acccgcacca
   51 ggtgcagacc ctgcgagtgt ggcggtaaac atattaggaa ccagcctgtg
   101 atgctggatg tgaccgagga gctgaggccc gatcacttgg tgctggcctg
   151 cacccgcgct gagtttggct ctagcgatga agatacagat tgaggtactg
   201 aaatggaatt ccggtc
<210> 6
   <211> 32
   <212> DNA
   <213> künstliche Sequenz
   <220>
   <223> Oligonukleotid 27635 zur PCR-Amplifikation der Sequenz bp 3328 - 3521 des humanen Adenovirus Typ 5
<400> 6
   gacgccaatt ccatttcagt acctcaatct gt 32
<210> 7
   <211> 31
   <212> DNA
   <213> künstliche Sequenz
   <220>
   <223> Oligonukleotid 27636 zur PCR-Amplifikation der Sequenz bp 3328 - 3521 des humanen Adenovirus Typ 5
<400> 7
   ctcgcggatc cagatctgga aggtgctgag g                    31
<210> 8
   <211> 782
   <212> DNA
   <213> Simian virus 40
   <223> 3'-Spleiß- und Polyadenylierungssignal von SV40 aus Plasmid pGL2basic, entsprechend bp 1978-2749, Genbank X65323
<400> 8
   1    cgactgaatt caatttttaa gtgtataatg tgttaaacta ctgattctaa
   51   ttgtttgtgt attttagatt ccaacctatg gaactgatga atgggagcag
   101  tggtggaatg cctttaatga ggaaaacctg ttttgctcag aagaaatgcc
   151  atctagtgat gatgaggcta ctgctgactc tcaacattct actcctccaa
   201  aaaagaagag aaaggtagaa gaccccaagg actttccttc agaattgcta
   251  agttttttga gtcatgctgt gtttagtaat agaactcttg cttgctttgc
   301  tatttacacc acaaaggaaa aagctgcact gctatacaag aaaattatgg
   351  aaaaatattc tgtaaccttt ataagtaggc ataacagtta taatcataac
   401  atactgtttt ttcttactcc acacaggcat agagtgtctg ctattaataa
   451  ctatgctcaa aaattgtgta cctttagctt tttaatttgt aaaggggtta
   501  ataaggaata tttgatgtat agtgccttga ctagagatca taatcagcca
   551  taccacattt gtagaggttt tacttgcttt aaaaaacctc ccacacctcc
   601  ccctgaacct gaaacataaa atgaatgcaa ttgttgttgt taacttgttt
   651  attgcagctt ataatggtta caaataaagc aatagcatca caaatttcac
   701  aaataaagca tttttttcac tgcattctag ttgtggtttg tccaaactca
   751  tcaatgtatc ttatcatgtc tggatccgtc ga
<210> 9
   <211> 32
   <212> DNA
   <213> künstliche Sequenz
   <220>
   <223> Oligonukleotid 27637 zur PCR-Amplifikation der Sequenz bp 1978 - 2748 des Plasmids pGL2basic
<400> 9
   cgactgaatt caatttttaa gtgtataatg tg 32
<210> 10
   <211> 27
   <212> DNA
   <213> künstliche Sequenz
   <220>
   <223> Oligonukleotid 27638 zur PCR-Amplifikation der Sequenz bp 1978 - 2748 des Plasmids pGL2basic
<400> 10
   tcgacggatc cagacatgat aagatac 27
<210> 11
   <211> 20
   <212> DNA
   <213> künstliche Sequenz
   <220>
   <223> Oligonukleotid 28231 (bp 901-920 des humanen Adenovirus Typ 5) zur Sequenzbestimmung des Plasmids STK146
<400> 11
   ccttgtaccg gaggtgatcg 20
<210> 12
   <211> 20
   <212> DNA
   <213> künstliche Sequenz
   <220>
   <223> Oligonukleotid 28232 (bp 1301-1320 des humanen Adenovirus Typ 5) zur Sequenzbestimmung des Plasmids STK146
<400> 12
   tggcgcctgc tatcctgaga 20
<210> 13
   <211> 20
   <212> DNA
   <213> künstliche Sequenz
   <220>
   <223> Oligonukleotid 28233 (bp 1701-1720 des humanen Adenovirus Typ 5) zur Sequenzbestimmung des Plasmids STK146
<400> 13
   tacatctgac ctcatggagg 20
<210> 14
   <211> 20
   <212> DNA
   <213> künstliche Sequenz
   <220>
   <223> Oligonukleotid 28234 (bp 2100-2119 des humanen Adenovirus Typ 5) zur Sequenzbestimmung des Plasmids STK146
<400> 14
   caagaatcgc ctgctactgt 20
<210> 15
   <211> 20
   <212> DNA
   <213> künstliche Sequenz
   <220>
   <223> Oligonukleotid 28235 (bp 2500-2519 des humanen Adenovirus Typ 5) zur Sequenzbestimmung des Plasmids STK146
<400> 15
   ggctgcagcc aggggatgat 20
<210> 16
   <211> 20
   <212> DNA
   <213> künstliche Sequenz
   <220>
   <223> Oligonukleotid 28236 (bp 2853-2972 des humanen Adenovirus Typ 5) zur Sequenzbestimmung des Plasmids STK146
<400> 16
   agggttcggg gctgtgcctt 20
<210> 17
   <211> 20
   <212> DNA
   <213> künstliche Sequenz
   <220>
   <223> Oligonukleotid 28237 (bp 2853-2872 des humanen Adenovirus Typ 5) zur Sequenzbestimmung des Plasmids STK146
<400> 17
   cctgaacggg gtgtttgaca 20
<210> 18
   <211> 7090
   <212> DNA
   <213> künstliche Sequenz
   <220>
   <223> Plasmid STK146
<400> 18
   1 gtggcacttt tcggggaaat gtgcgcggaa cccctatttg tttatttttc
   51 taaatacatt caaatatgta tccgctcatg agacaataac cctgataaat
   101 gcttcaataa tattgaaaaa ggaagagtat gagtattcaa catttccgtg
   151 tcgcccttat tccctttttt gcggcatttt gccttcctgt ttttgctcac
   201 ccagaaacgc tggtgaaagt aaaagatgct gaagatcagt tgggtgcacg
   251 agtgggttac atcgaactgg atctcaacag cggtaagatc cttgagagtt
   301 ttcgccccga agaacgtttt ccaatgatga gcacttttaa agttctgcta
   351 tgtggcgcgg tattatcccg tattgacgcc gggcaagagc aactcggtcg
   401 ccgcatacac tattctcaga atgacttggt tgagtactca ccagtcacag
   451 aaaagcatct tacggatggc atgacagtaa gagaattatg cagtgctgcc
   501 ataaccatga gtgataacac tgcggccaac ttacttctga caacgatcgg
   551 aggaccgaag gagctaaccg cttttttgca caacatgggg gatcatgtaa
   601 ctcgccttga tcgttgggaa ccggagctga atgaagccat accaaacgac
   651 gagcgtgaca ccacgatgcc tgtagcaatg gcaacaacgt tgcgcaaact
   701 attaactggc gaactactta ctctagcttc ccggcaacaa ttaatagact
   751 ggatggaggc ggataaagtt gcaggaccac ttctgcgctc ggcccttccg
   801 gctggctggt ttattgctga taaatctgga gccggtgagc gtgggtctcg
   851 cggtatcatt gcagcactgg ggccagatgg taagccctcc cgtatcgtag
   901 ttatctacac gacggggagt caggcaacta tggatgaacg aaatagacag
   951 atcgctgaga taggtgcctc actgattaag cattggtaac tgtcagacca
   1001 agtttactca tatatacttt agattgattt aaaacttcat ttttaattta
   1051 aaaggatcta ggtgaagatc ctttttgata atctcatgac caaaatccct
   1101 taacgtgagt tttcgttcca ctgagcgtca gaccccgtag aaaagatcaa
   1151 aggatcttct tgagatcctt tttttctgcg cgtaatctgc tgcttgcaaa
   1201 caaaaaaacc accgctacca gcggtggttt gtttgccgga tcaagagcta
   1251 ccaactcttt ttccgaaggt aactggcttc agcagagcgc agataccaaa
   1301 tactgtcctt ctagtgtagc cgtagttagg ccaccacttc aagaactctg
   1351 tagcaccgcc tacatacctc gctctgctaa tcctgttacc agtggctgct
   1401 gccagtggcg ataagtcgtg tcttaccggg ttggactcaa gacgatagtt
   1451 accggataag gcgcagcggt cgggctgaac ggggggttcg tgcacacagc
   1501 ccagcttgga gcgaacgacc tacaccgaac tgagatacct acagcgtgag
   1551 ctatgagaaa gcgccacgct tcccgaaggg agaaaggcgg acaggtatcc
   1601 ggtaagcggc agggtcggaa caggagagcg cacgagggag cttccagggg
   1651 gaaacgcctg gtatctttat agtcctgtcg ggtttcgcca cctctgactt
   1701 gagcgtcgat ttttgtgatg ctcgtcaggg gggcggagcc tatggaaaaa
   1751 cgccagcaac gcggcctttt tacggttcct ggccttttgc tggccttttg
   1801 ctcacatgtt ctttcctgcg ttatcccctg attctgtgga taaccgtatt
   1851 accgcctttg agtgagctga taccgctcgc cgcagccgaa cgaccgagcg
   1901 cagcgagtca gtgagcgagg aagcggaaga gcgcccaata cgcaaaccgc
   1951 ctctccccgc gcgttggccg attcattaat gcagctggca cgacaggttt
   2001 cccgactgga aagcgggcag tgagcgcaac gcaattaatg tgagttagct
   2051 cactcattag gcaccccagg ctttacactt tatgcttccg gctcgtatgt
   2101 tgtgtggaat tgtgagcgga taacaatttc acacaggaaa cagctatgac
   2151 catgattacg ccaagcgcgc aattaaccct cactaaaggg aacaaaagct
   2201 gggtaccggg ccccccctcg aggtcatcga attctaccgg gtaggggagg
   2251 cgcttttccc aaggcagtct ggagcatgcg ctttagcagc cccgctggca
   2301 cttggcgcta cacaagtggc ctctggcctc gcacacattc cacatccacc
   2351 ggtaggcgcc aaccggctcc gttctttggt ggccccttcg cgccaccttc
   2401 tactcctccc ctagtcagga agttcccccc cgccccgcag ctcgcgtcgt
   2451 gcaggacgtg acaaatggaa gtagcacgtc tcactagtct cgtgcagatg
   2501 gacagcaccg ctgagcaatg gaagcgggta ggcctttggg gcagcggcca
   2551 atagcagctt tgctccttcg ctttctgggc tcagaggctg ggaaggggtg
   2601 ggtccggggg cgggctcagg ggcgggctca ggggcggggc gggcgcccga
   2651 aggtcctccg gaggcccggc attctcgcac gcttcaaaag cgcacgtctg
   2701 ccgcgctgtt ctcctcttcc tcatctccgg gcctttcgac cagcttgata
   2751 tcgagtgcca gcgagtagag ttttctcctc cgagccgctc cgacaccggg
   2801 actgaaaatg agacatatta tctgccacgg aggtgttatt accgaagaaa
   2851 tggccgccag tcttttggac cagctgatcg aagaggtact ggctgataat
   2901 cttccacctc ctagccattt tgaaccacct acccttcacg aactgtatga
   2951 tttagacgtg acggcccccg aagatcccaa cgaggaggcg gtttcgcaga
   3001 tttttcccga ctctgtaatg ttggcggtgc aggaagggat tgacttactc
   3051 acttttccgc cggcgcccgg ttctccggag ccgcctcacc tttcccggca
   3101 gcccgagcag ccggagcaga gagccttggg tccggtttct atgccaaacc
   3151 ttgtaccgga ggtgatcgat cttacctgcc acgaggctgg ctttccaccc
   3201 agtgacgacg aggatgaaga gggtgaggag tttgtgttag attatgtgga
   3251 gcaccccggg cacggttgca ggtcttgtca ttatcaccgg aggaatacgg
   3301 gggacccaga tattatgtgt tcgctttgct atatgaggac ctgtggcatg
   3351 tttgtctaca gtaagtgaaa attatgggca gtgggtgata gagtggtggg
   3401 tttggtgtgg taattttttt tttaattttt acagttttgt ggtttaaaga
   3451 attttgtatt gtgatttttt taaaaggtcc tgtgtctgaa cctgagcctg
   3501 agcccgagcc agaaccggag cctgcaagac ctacccgccg tcctaaaatg
   3551 gcgcctgcta tcctgagacg cccgacatca cctgtgtcta gagaatgcaa
   3601 tagtagtacg gatagctgtg actccggtcc ttctaacaca cctcctgaga
   3651 tacacccggt ggtcccgctg tgccccatta aaccagttgc cgtgagagtt
   3701 ggtgggcgtc gccaggctgt ggaatgtatc gaggacttgc ttaacgagcc
   3751 tgggcaacct ttggacttga gctgtaaacg ccccaggcca taaggtgtaa
   3801 acctgtgatt gcgtgtgtgg ttaacgcctt tgtttgctga atgagttgat
   3851 gtaagtttaa taaagggtga gataatgttt aacttgcatg gcgtgttaaa
   3901 tggggcgggg cttaaagggt atataatgcg ccgtgggcta atcttggtta
   3951 catctgacct catggaggct tgggagtgtt tggaagattt ttctgctgtg
   4001 cgtaacttgc tggaacagag ctctaacagt acctcttggt tttggaggtt
   4051 tctgtggggc tcatcccagg caaagttagt ctgcagaatt aaggaggatt
   4101 acaagtggga atttgaagag cttttgaaat cctgtggtga gctgtttgat
   4151 tctttgaatc tgggtcacca ggcgcttttc caagagaagg tcatcaagac
   4201 tttggatttt tccacaccgg ggcgcgctgc ggctgctgtt gcttttttga
   4251 gttttataaa ggataaatgg agcgaagaaa cccatctgag cggggggtac
   4301 ctgctggatt ttctggccat gcatctgtgg agagcggttg tgagacacaa
   4351 gaatcgcctg ctactgttgt cttccgtccg cccggcgata ataccgacgg
   4401 aggagcagca gcagcagcag gaggaagcca ggcggcggcg gcaggagcag
   4451 agcccatgga acccgagagc cggcctggac cctcgggaat gaatgttgta
   4501 caggtggctg aactgtatcc agaactgaga cgcattttga caattacaga
   4551 ggatgggcag gggctaaagg gggtaaagag ggagcggggg gcttgtgagg
   4601 ctacagagga ggctaggaat ctagctttta gcttaatgac cagacaccgt
   4651 cctgagtgta ttacttttca acagatcaag gataattgcg ctaatgagct
   4701 tgatctgctg gcgcagaagt attccataga gcagctgacc acttactggc
   4751 tgcagccagg ggatgatttt gaggaggcta ttagggtata tgcaaaggtg
   4801 gcacttaggc cagattgcaa gtacaagatc agcaaacttg taaatatcag
   4851 gaattgttgc tacatttctg ggaacggggc cgaggtggag atagatacgg
   4901 aggatagggt ggcctttaga tgtagcatga taaatatgtg gccgggggtg
   4951 cttggcatgg acggggtggt tattatgaat gtaaggttta ctggccccaa
   5001 ttttagcggt acggttttcc tggccaatac caaccttatc ctacacggtg
   5051 taagcttcta tgggtttaac aatacctgtg tggaagcctg gaccgatgta
   5101 agggttcggg gctgtgcctt ttactgctgc tggaaggggg tggtgtgtcg
   5151 ccccaaaagc agggcttcaa ttaagaaatg cctctttgaa aggtgtacct
   5201 tgggtatcct gtctgagggt aactccaggg tgcgccacaa tgtggcctcc
   5251 gactgtggtt gcttcatgct agtgaaaagc gtggctgtga ttaagcataa
   5301 catggtatgt ggcaactgcg aggacagggc ctctcagatg ctgacctgct
   5351 cggacggcaa ctgtcacctg ctgaagacca ttcacgtagc cagccactct
   5401 cgcaaggcct ggccagtgtt tgagcataac atactgaccc gctgttcctt
   5451 gcatttgggt aacaggaggg gggtgttcct accttaccaa tgcaatttga
   5501 gtcacactaa gatattgctt gagcccgaga gcatgtccaa ggtgaacctg
   5551 aacggggtgt ttgacatgac catgaagatc tggaaggtgc tgaggtacga
   5601 tgagacccgc accaggtgca gaccctgcga gtgtggcggt aaacatatta
   5651 ggaaccagcc tgtgatgctg gatgtgaccg aggagctgag gcccgatcac
   5701 ttggtgctgg cctgcacccg cgctgagttt ggctctagcg atgaagatac
   5751 agattgaggt actgaaatgg aattcctcta gtgatgatga ggctactgct
   5801 gactctcaac attctactcc tccaaaaaag aagagaaagg tagaagaccc
   5851 caaggacttt ccttcagaat tgctaagttt tttgagtcat gctgtgttta
   5901 gtaatagaac tcttgcttgc tttgctattt acaccacaaa ggaaaaagct
   5951 gcactgctat acaagaaaat tatggaaaaa tattctgtaa cctttataag
   6001  taggcataac agttataatc ataacatact gttttttctt actccacaca
   6051  ggcatagagt gtctgctatt aataactatg ctcaaaaatt gtgtaccttt
   6101  agctttttaa tttgtaaagg ggttaataag gaatatttga tgtatagtgc
   6151  cttgactaga gatcataatc agccatacca catttgtaga ggttttactt
   6201  gctttaaaaa acctcccaca cctccccctg aacctgaaac ataaaatgaa
   6251  tgcaattgtt gttgttaact tgtttattgc agcttataat ggttacaaat
   6301  aaagcaatag catcacaaat ttcacaaata aagcattttt ttcactgcat
   6351  tctagttgtg gtttgtccaa actcatcaat gtatcttatc atgtctggat
   6401  ccactagttc tagagcggcc gccaccgcgg tggagctcca attcgcccta
   6451  tagtgagtcg tattacgcgc gctcactggc cgtcgtttta caacgtcgtg
   6501  actgggaaaa ccctggcgtt acccaactta atcgccttgc agcacatccc
   6551  cctttcgcca gctggcgtaa tagcgaagag gcccgcaccg atcgcccttc
   6601  ccaacagttg cgcagcctga atggcgaatg ggacgcgccc tgtagcggcg
   6651  cattaagcgc ggcgggtgtg gtggttacgc gcagcgtgac cgctacactt
   6701  gccagcgccc tagcgcccgc tcctttcgct ttcttccctt cctttctcgc
   6751  cacgttcgcc ggctttcccc gtcaagctct aaatcggggg ctccctttag
   6801  ggttccgatt tagtgcttta cggcacctcg accccaaaaa acttgattag
   6851  ggtgatggtt cacgtagtgg gccatcgccc tgatagacgg tttttcgccc
   6901  tttgacgttg gagtccacgt tctttaatag tggactcttg ttccaaactg
   6951  gaacaacact caaccctatc tcggtctatt cttttgattt ataagggatt
   7001  ttgccgattt cggcctattg gttaaaaaat gagctgattt aacaaaaatt
   7051  taacgcgaat tttaacaaaa tattaacgct tacaatttag